# EUROPEAN PATENT SPECIFICATION

(11) **EP 3 302 594 B1**
(45) Date of publication and mention of the grant of the patent: **11.09.2024**
(21) Application number: 16732064.7
(22) Date of filing: 03.06.2016
(51) Int. Cl.: A61K 38/39, A61P 17/02, A61P 3/10, A61P 31/04, A61P 31/12, A61P 43/00, A61P 9/00, A61P 9/04, A61P 9/10, A61L 27/36, A61L 27/38

(54) **CARDIAC FIBROBLAST-DERIVED EXTRACELLULAR MATRIX AND INJECTABLE FORMULATIONS THEREOF FOR TREATMENT OF ISCHEMIC DISEASE OR INJURY**
HERZFIBROBLASTEN-ABGELEITETE EXTRAZELLULÄRE MATRIX UND INJIZIERBARE FORMULIERUNGEN DAVON ZUR BEHANDLUNG VON ISCHÄMISCHER ERKRANKUNG ODER VERLETZUNG
MATRICE EXTRACELLULAIRE DÉRIVÉE DE FIBROBLASTES CARDIAQUES ET FORMULATIONS INJECTABLES CORRESPONDANTES POUR LE TRAITEMENT D'UNE MALADIE OU D'UNE LÉSION ISCHÉMIQUE

(30) Priority: 03.06.2015 US 201562170324 P
(43) Date of publication of application: 11.04.2018
(73) Proprietor: Wisconsin Alumni Research Foundation, Madison, WI 53726 (US)
(72) Inventor: SCHMUCK, Eric, Sun Prairie, WI 53590 (US); RAVAL, Amish, Middleton, WI 53562 (US)
(74) Representative: J A Kemp LLP
(86) International application number: PCT/US2016/035881
(87) International publication number: WO 2016/197038

(56) References cited:
- WO-A1-2013/028968
- US-A1- 2007 014 773

## Description

### BACKGROUND

Ischemia is an interruption in the arterial blood flow to a tissue, organ, or extremity. Ischemia causes a shortage of oxygen and glucose needed for cellular metabolism, and thus can result in tissue damage or death (ischemic injury).

Cardiac ischemia, which can result in myocardial infarction, occurs when the myocardium (heart muscle) receives insufficient blood flow. Ischemic injury to the heart is a major cause of hospital admissions and death worldwide, and new and more effective treatments for cardiac ischemic injury are needed to improve patient outcomes.

Limb ischemia, which has an etiology and standard treatment regimen that is distinct from cardiac ischemia, is the result of lack of blood flow to a limb. In the United States, about two million people annually suffer from limb ischemia. Upon presentation, 60% of these patients have no good treatment options available. Ischemic ulcers, which are a common form of ischemic limb injury, are chronic, non-healing painful wounds that are prone to repeated infections. Often, there is no medication that can successfully treat ischemic limb injury. Revascularization of the tissue and/or the use of various skin substitutes are common treatments, but these treatments are not always sufficient to salvage the injured limb, resulting in amputation as the only viable treatment option.

In a previous patent (U.S. Patent No. 8,802,144) and publication (Schmuck, E.G., et al., Cardiovasc Eng Technol 5(1) (2014): 119-131) Schmuck et al. describe a bioscaffold made from an engineered cardiac fibroblast-derived extracellular matrix that can be used as a platform to transfer therapeutic cells to injured or diseased heart tissue. The bioscaffold is seeded with therapeutic cells as a mechanism to deliver the therapeutic cells to the injured or diseased heart tissue. This therapeutic method requires performing invasive and potentially high-risk surgery to place the cell-seeded patch onto a surface of the injured heart. Furthermore, this therapeutic method requires the use of therapeutic cells that are not endogenous to the patient, adding further complexity, potential risk, and additional regulatory hurdles. Finally, this therapeutic method is specific to injuries and diseases of the heart tissue, and would not be expected to work with injuries occurring in non-cardiac tissue, such as with ischemic limb injuries.

US 2007/014773 which is concerned with compositions for regenerating defective or absent myocardium.

WO2013/028968 A1 discloses a bioscaffold for facilitating the delivery of cells to myocardial tissue comprising an isolated 3-dimensional cardiac extracellular matrix derived from cardiac fibroblast cells, wherein the cardiac extracellular matrix comprises the structural proteins fibronectin, collagen type 1.

For these reasons, new compositions and methods of using the previously disclosed bioscaffold made from an engineered cardiac fibroblast-derived extracellular matrix would be highly desirable.

### BRIEF SUMMARY

The inventors demonstrate herein that injectable compositions made from the engineered cardiac fibroblast-derived extracellular matrix described in US. Patent No. 8,802,144 can be made and delivered into the heart. Furthermore, the inventors demonstrate herein that the engineered cardiac fibroblast-derived extracellular matrix, whether seeded with therapeutic cells or not, can effectively treat ischemic limb injury. Finally, the inventors demonstrate herein that even in the absence of therapeutic cells, the engineered cardiac fibroblast-derived extracellular matrix can effectively treat both ischemic cardiac and ischemic limb injury. The present invention is limited to the scope of the appended claims.

Accordingly, in a first aspect, the present invention provides an injectable composition for treating cardiac disease, cardiac injury, or ischemic injury, comprising:
(a) fragments of an engineered cardiac fibroblast cell-derived extracellular matrix (CF-ECM) comprising the structural proteins fibronectin, collagen type I, collagen type III, and elastin, wherein from 60% to 90% of the structural proteins present in the engineered extracellular matrix are fibronectin, wherein the one or more fragments are small enough to pass through the injection opening of an 18-gauge hypodermic needle; and
(b) an injectable pharmaceutically acceptable carrier, wherein the fragments are suspended in the carrier.

In some embodiments, the structural proteins of the engineered cardiac extracellular matrix are not chemically cross-linked.

In some embodiments, the one or more fragments have a thickness of 20-500 µm.

In some embodiments, the one or more fragments are in the form of a lyophilized powder.

In some embodiments, the engineered cardiac extracellular matrix further comprises one or more of the matricellular proteins latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, or biglycan.

In some embodiments, the formulation is essentially devoid of intact cardiac fibroblast cells. In some such embodiments, the formulation is completely cell free.

In some embodiments, the composition further includes one or more cells that are therapeutic for cardiac disease, cardiac injury, or ischemic injury. In some such embodiments, the one or more cells may include skeletal myoblasts, embryonic stem (ES) cells or derivatives thereof, induced pluripotent stem (iPS) cells or derivatives thereof, multipotent adult germline stem (maGCS) cells, bone marrow mesenchymal stem cells (BMSCs), very small embryonic-like stem cells (VSEL cells), endothelial progenitor cells (EPCs), cardiopoietic cells (CPCs), cardiosphere-derived cells (CDCs), multipotent *Isl1*⁺ cardiovascular progenitor cells (MICPs), epicardium-derived progenitor cells (EPDCs), adipose-derived stem cells, human mesenchymal stem cells derived from iPS or ES cells, human mesenchymal stromal cells derived from iPS or ES cells, or combinations thereof.

In some embodiments, the composition further includes one or more exogenous non-cellular therapeutic agents. In some such embodiments, the one or more exogenous non-cellular therapeutic agents may include one or more growth factors. In some such embodiments, the one or more growth factors may include stromal cell-derived factor 1 (SDF-1), epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), or keratinocyte growth factor (KGF).

In some embodiments, the one or more fragments are small enough to pass through the injection opening of a 27-gauge hypodermic needle.

In some embodiments, the cardiac disease, cardiac injury, or ischemic disease or injury is caused by an acute myocardial infarct, heart failure, viral infection, bacterial infection, congenital defect, stroke, diabetic foot ulcers, peripheral artery disease (PAD), PAD-associated ulcers, or limb ischemia.

Also described, but not part of the invention, is a method for preparing an injectable composition. The method includes the steps of (a) obtaining a decellularized engineered cardiac fibroblast cell-derived extracellular matrix having a thickness of 20-500 µm and comprising the structural proteins fibronectin, collagen type I, collagen type III, and elastin, wherein from 60% to 90% of the structural proteins present in the engineered extracellular matrix are fibronectin; (b) either (i) dicing the engineered cardiac fibroblast cell-derived extracellular matrix into fragments sufficiently small to be capable of passing through the injection opening of an 18 gauge hypodermic needle, or (ii) lyophilizing the engineered cardiac fibroblast cell-derived extracellular matrix into a powder; and (c) mixing the diced or lyophilized engineered cardiac fibroblast cell-derived extracellular matrix with an injectable pharmaceutically acceptable carrier.

In some embodiments, the method further includes the step of passing the resulting composition through one or more passages having a minimum flow area of less than 0.60 mm². By "minimum flow area," we meant the minimum cross sectional area of the passage, as measured on a cross section that is perpendicular to the direction of flow through the passage. For example, if the passage is a standard hypodermic needle, the minimum flow area would be the area of the circle that defines a cross section of the inside surface of the hypodermic needle. A standard gauge 18 hypodermic needle having an inner diameter of .838 mm would have a minimum flow area of .552 mm², based on the area of a circle formula (cross sectional area = πr²). In some such embodiments, at least one of the one or more passages has a minimum flow area of less than 0.040 mm².

In some embodiments, the step of obtaining the engineered cardiac fibroblast cell-derived extracellular matrix is performed by (a) isolating cardiac fibroblasts from cardiac tissue or deriving cardiac fibroblasts from induced pluripotent stem (iPS) cells; (b) expanding the cardiac fibroblasts in culture for 1-15 passages; and (c) plating the expanded cardiac fibroblasts into a culture having a cell density of 100,000 to 500,000 cells per cm². In performing these steps, the cardiac fibroblasts secrete a 3-dimensional cardiac fibroblast derived extracellular matrix (CF-ECM) having a thickness of 20-500 µm. In some such embodiments, this step further includes contacting the CF-ECM with a decellularizing agent, whereby the cardiac extracellular matrix is decellularized.

In some embodiments, the method further includes the step of adding to the injectable composition one or more cells that are therapeutic for cardiac disease, cardiac injury, or ischemic injury. In some such embodiments, the one or more cells may include skeletal myoblasts, embryonic stem (ES) cells or derivatives thereof, induced pluripotent stem (iPS) cells or derivatives thereof, multipotent adult germline stem cells (maGCSs), bone marrow mesenchymal stem cells (BMSCs), very small embryonic-like stem cells (VSEL cells), endothelial progenitor cells (EPCs), cardiopoietic cells (CPCs), cardiosphere-derived cells (CDCs), multipotent *Isl1*⁺ cardiovascular progenitor cells (MICPs), epicardium-derived progenitor cells (EPDCs), adipose-derived stem cells, human mesenchymal stem cells derived from iPS or ES cells, human mesenchymal stromal cells derived from iPS or ES cells, or combinations thereof.

In some embodiments, the method further includes the step of adding to the injectable composition one or more non-cellular therapeutic agents. In some such embodiments, the one or more non-cellular therapeutic agents may include one or more growth factors. In some such embodiments, the one or more growth factors may include stromal cell-derived factor 1 (SDF-1), epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), or keratinocyte growth factor (KGF).

The present invention also provides a cell free engineered cardiac fibroblast cell-derived extracellular matrix for use in a method for treating a subject having an ischemic disease or injury, the method comprising injecting the injectable composition of any one of claims 1 to 6 into a tissue of the subject that is exhibiting an ischemic disease or injury, wherein no cells are seeded onto the engineered cardiac fibroblast cell-derived extracellular matrix, whereby the severity of the ischemic disease or injury is decreased.

In some embodiments, the cell free engineered cardiac extracellular matrix includes one or more of the matricellular proteins latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, and biglycan.

In some embodiments, the cell free engineered cardiac fibroblast cell-derived extracellular matrix is obtained by (a) isolating cardiac fibroblasts from cardiac tissue or deriving cardiac fibroblasts from induced pluripotent stem (iPS) cells; (b) expanding the cardiac fibroblasts in culture for 1-15 passages; (c) plating the expanded cardiac fibroblasts into a culture having a cell density of 100,000 to 500,000 cells per cm², wherein the cardiac fibroblasts secrete a 3-dimensional cardiac extracellular matrix having a thickness of 20-500 µm; and (d) contacting the cardiac extracellular matrix with a decellularizing agent, whereby the cardiac extracellular matrix is decellularized.

In some embodiments, engineered cardiac fibroblast cell-derived extracellular matrix for use wherein the method further includes contacting the tissue of the subject that is exhibiting an ischemic disease or injury with one or more exogenous non-cellular therapeutic agents. In some such embodiments, the one or more non-cellular therapeutic agents include one or more growth factors. In some such embodiments, the one or more growth factors may include stromal cell-derived factor 1 (SDF-1), epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), or keratinocyte growth factor (KGF).

In some embodiments, the ischemic disease or injury is caused by myocardial infarct, stroke, diabetic foot ulcers, peripheral artery disease (PAD), PAD-associated ulcers, or limb ischemia. In some such embodiments, the ischemic disease or injury is caused by limb ischemia. In some such embodiments, the limb ischemia is the result of atherosclerosis or diabetes.

The invention further provides an engineered cardiac fibroblast cell-derived extracellular matrix for use in a method for treating a subject having an ischemic limb injury, the method comprising injecting into a tissue of the subject that is exhibiting an ischemic limb injury the fragments of engineered cardiac fibroblast cell-derived extracellular matrix, the fragments having a thickness of 20-500 µm comprising the structural proteins fibronectin, collagen type I, collagen type III, and elastin, wherein from 60% to 90% of the structural proteins present in the engineered extracellular matrix are fibronectin, wherein the engineered cardiac fibroblast cell-derived extracellular matrix is fragmented and suspended in an isotonic solution to form an injectable suspension and is then administered to the subject by injection, whereby the severity of the ischemic limb injury is decreased.

In some embodiments, the engineered cardiac extracellular matrix includes one or more of the matricellular proteins latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, or biglycan.

In some embodiments, the engineered cardiac extracellular matrix is seeded with one or more cells that are therapeutic for ischemic limb injury. In some such embodiments, the one or more cells that are therapeutic for ischemic limb injury may include skeletal myoblasts, embryonic stem (ES) cells or derivatives thereof, induced pluripotent stem (iPS) cells or derivatives thereof, multipotent adult germline stem cells (maGCSs), bone marrow mesenchymal stem cells (BMSCs), very small embryonic-like stem cells (VSEL cells), endothelial progenitor cells (EPCs), adipose-derived stem cells, human mesenchymal stem cells derived from iPS or ES cells, human mesenchymal stromal cells derived from iPS or ES cells, or combinations thereof.

In some embodiments, the engineered cardiac fibroblast cell-derived extracellular matrix is obtained by (a) isolating cardiac fibroblasts from cardiac tissue or deriving cardiac fibroblasts from induced pluripotent stem (iPS) cells; (b) expanding the cardiac fibroblasts in culture for 1-15 passages; and (c) plating the expanded cardiac fibroblasts into a culture having a cell density of 100,000 to 500,000 cells per cm², wherein the cardiac fibroblasts secrete a 3-dimensional cardiac extracellular matrix having a thickness of 20-500 µm.

In some embodiments, the engineered cardiac fibroblast cell-derived extracellular matrix is for use in a method that further includes contacting the tissue of the subject that is exhibiting an ischemic injury with one or more exogenous non-cellular therapeutic agents. In some such embodiments, the one or more non-cellular therapeutic agents may include one or more growth factors. In some such embodiments, the one or more growth factors may include stromal cell-derived factor 1 (SDF-1), epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), or keratinocyte growth factor (KGF).

In some embodiments, the ischemic limb injury is the result of atherosclerosis or diabetes.

The disclosed compositions and methods are further detailed below. Please note that reference herein to methods of treatment practiced on the human or animal body should be taken as describing the injectable compositions and cell free engineered cardiac fibroblast cell-derived extracellular matrix provided for use in such methods, rather than the methods themselves being provided.

### BRIEF DESCRIPTION OF DRAWINGS

The invention will be better understood and features, aspects, and advantages other than those set forth above will become apparent when consideration is given to the following detailed description thereof. Such detailed description makes reference to the following drawings.

The patent or application file contains at least one drawing executed in color. Copies of this patent or patent application publication with color drawing(s) will be provided by the Office upon request and payment of the necessary fee.
FIG. 1 shows fragmented CF-ECM stained with black tissue dye. The fragmentation protocol results in heterogenous CF-ECM fragments. Fragments appear as small "sheets" of CF-ECM.
FIG. 2 shows fragmented CF-ECM stained with black tissue dye.
FIG. 3 shows fragmented CF-ECM stained with black tissue dye.
FIG. 4 shows fragmented CF-ECM stained with black tissue dye.
FIG. 5 shows CF-ECM injectable formulation colored with black tissue dye and injected via cardiac catheter into a porcine LV. Note how the CF-ECM lodges in the interstitial space.
FIG. 6 is a graph showing change of ejection fraction for the four different treatment groups at various times after MI, as measured by echocardiogram. The patch only treatment stabilized change at -10%.
FIG. 7 is a graph showing end systolic volume for the four different treatment groups at various times after MI, as measured by echocardiogram.
FIG. 8 is a graph showing change in end systolic volume at day 28 post MI for the four different treatment groups.
FIG. 9 are graphs showing cross section pathology measurements for the four different treatment groups. The measurements shown are scar thickness (upper left panel), hinge point thickness (upper right panel), remote wall thickness (lower left panel), and % left ventricle infarcted (lower right panel).

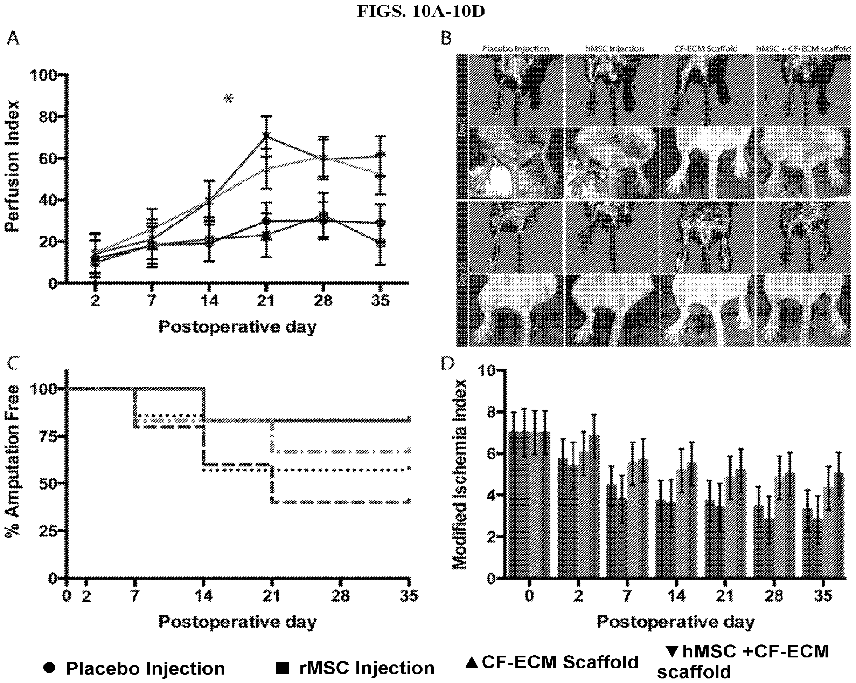

FIG. 10A is a graph showing Perfusion Index as a function of days post-operative for the four treatment groups in the hind limb ischemia model. Specifically, mice received treatment after double ligation of the femoral artery with CF-ECM loaded with 1.0 × 10⁶ GFP+ MSC's, CF-ECM only without cells, GFP+ MSC only delivered by intra-muscular injection, or placebo. Perfusion Index is calculated by comparing blood flow, by scanning laser Doppler imaging, in the affected limb to the normal limb. Animals treated with CF-ECM had significantly greater blood flow than cell treated and placebo controls (p=0.003).
FIG. 10B shows representative images of laser Doppler scans and corresponding photographs for the four treatment groups in the hind limb ischemia model.
FIG. 10C is a graph showing freedom from autoamputation as a function of days post-operative for the four treatment groups in the hind limb ischemia model. CF-ECM treated animals had greater foot retention than cell and placebo treated animals.
FIG. 10D is a graph of Modified Ischemia Index as a function of days post-operative for the four treatment groups in the hind limb ischemia model. The Modified Ischemic Index is an overall assessment of limb health. Modified Ischemic Index showed a strong trend toward improvement in the CF-ECM treated animals compared to the cell treated and placebo controls.
FIG. 11 shows representative Hematoxylin and Eosin staining of affected calf and thigh muscles for each treatment group. Quantitative histological scoring of affected tissues is shown in Table 1.
FIG. 12 presents data for GFP+ mesenchymal stem cells (MSCs) transferred with CF-ECM in a mouse hindlimb ischemia model.
FIG. 13 demonstrates that GFP+ MSCs transferred with CF-ECM decreased post-myocardial infarction (MI) remodeling.
FIG. 14 demonstrates that GFP+ MSCs transferred with CF-ECM decreased post-MI remodeling.
FIG. 15 demonstrates that GFP+ MSCs transferred with CF-ECM decreased post-MI remodeling.
FIG. 16 demonstrates efficacy of administration of CF-ECM alone relative to sham treatment.
FIG. 17 demonstrates efficacy of administration of CF-ECM alone relative to sham treatment.
FIG. 18 shows the results of 1-hour seeding of MSCs into injectable CF-ECM.
FIG. 19 shows 18-hour seeding of MSCs into injectable CF-ECM.
FIG. 20 shows increased cell retention four (4) hours post injection of MSCs in combination with injectable ECM relative to injection of MSCs alone. Blue = MSC injection. Yellow = injectable CF-ECM + MSC injection.
FIG. 21 shows increased cell retention 24- and 48-hours post injection of MSCs in combination with injectable ECM.

### DETAILED DESCRIPTION

This application discloses an injectable formulation of a previously disclosed engineered cardiac fibroblast derived extracellular matrix (CF-ECM), as well as providing the formulation for use in methods as described herein. Such formulations are also provided for use in methods of treating cardiac disease or injury, and other ischemic injury, such an ischemic limb injury. Advantageously, this formulation can be delivered directly to the target tissue by injection, without the need for invasive surgery.

The application further discloses a cell free engineered CF-ECM that is not seeded with therapeutic cells for use in a method for for treating cardiac disease or injury, and the use of the CF-ECM, either with or without seeded therapeutic cells, for treating ischemic limb injury. The CF-ECM forms a patch that adheres well to the target tissue without the need for glue or sutures, moves flexibly, and successfully facilitates the delivery of seeded cells to the target tissue.

The engineered CF-ECM includes the structural proteins fibronectin, collagen type I, collagen type III, and elastin, and may include other structural proteins as well. In some embodiments, the engineered CF-ECM includes the structural protein collagen type V.

Fibronectin molecules make up from 60% to 90% of the structural protein molecules present in the engineered CF-ECM. In some embodiments, fibronectin molecules make up from 70% to 90% of the structural protein molecules present in the ECM. In some embodiments, fibronectin molecules make up from 80% to 90% of the structural protein molecules present in the engineered CF-ECM.

Before it is fragmented or lyophilized, the engineered CF-ECM has a thickness of 20-500 µm. In some embodiments, the unfragmented CF-ECM has a thickness range of 30-200 µm or of 50-150 µm. In some embodiments, more than 80% of the structural protein molecules present are fibronectin molecules.

Preferably, the structural proteins of the engineered CF-ECM are not chemically cross-linked.

In addition to the structural proteins, the cardiac ECM may include one or more matricellular proteins, such as growth factors and cytokines, as well as other substance. Non-limiting examples of other proteins that may be found in the cardiac ECM include latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, protein-lysine 6-oxidase, and biglycan. In some embodiments, the ECM may optionally include one or more of transforming growth factor beta 1 (TGFB-1), transforming growth factor beta 3 (TGFB-3), epidermal growth factor-like protein 8, growth/differentiation factor 6, granulins, galectin 3 binding protein, nidogen 1, nidogen 2, decorin, prolargin, vascular endothelial growth factor D (VEGF-D), Von Willebrand factor A1, Von Willebrand factor A5 A, matrix metalloprotease 14, matrix metalloprotease 23, platelet factor 4, prothrombin, tumor necrosis factor ligand superfamily member 11, and glia derived nexin.

Optionally, the engineered CF-ECM is decellularized, and is thus essentially devoid of intact cardiac fibroblast cells. In some embodiments, the CF-ECM may be seeded using methods that are known in the art with one or more cells that are therapeutic for cardiac disease or injury. Examples of therapeutic cells types that could be used to seed the bioscaffold include without limitation skeletal myoblasts, embryonic stem cells (ES), induced pluripotent stem cells (iPS), multipotent adult germline stem cells (maGCSs), bone marrow mesenchymal stem cells (BMSCs), very small embryonic-like stem cells (VSEL cells), endothelial progenitor cells (EPCs), cardiopoietic cells (CPCs), cardiosphere-derived cells (CDCs), multipotent *Isl1*⁺ cardiovascular progenitor cells (MICPs), epicardium-derived progenitor cells (EPDCs), adipose-derived stem cells, human mesenchymal stem cells (derived from iPS or ES cells), human mesenchymal stromal cells (derived from iPS or ES cells), or combinations thereof. All of these cell types are well-known in the art.

Methods of making the engineered CF-ECM and more information about its structure and composition are disclosed in, e.g., U.S. Patent No. 8,802,144.

The disclosed injectable compositions include one or more fragments of the engineered CF-ECMs, along with an injectable pharmaceutically acceptable carrier, where the fragments are sufficiently small to be able to freely pass through a hypodermic needle opening. In some embodiments, the fragments are sufficiently small to pass through the opening of a gauge 18 hypodermic needle having a nominal inner diameter of .838 mm. In other embodiments, the fragments are sufficiently small to pass through the opening of a gauge 19 (nominal inner diameter .686 mm), gauge 20 (nominal inner diameter .603 mm), gauge 21 (nominal inner diameter .514 mm), gauge 22 (nominal inner diameter .413 mm), gauge 23 (nominal inner diameter .337 mm), gauge 24 (nominal inner diameter .311 mm), gauge 25 (nominal inner diameter .260 mm), gauge 26 (nominal inner diameter .260 mm), and/or gauge 27 (nominal inner diameter .210 mm) hypodermic needle.

In a non-limiting example, the fragments may be formed by dicing the engineered CF-ECM, with, e.g., a razor blade, scalpel, or scissors. Optionally, once created, the fragments may be suspended and passed through a hypodermic needle one or more times.

In another non-limiting example, the fragments may be formed by lyophilizing (freeze-drying and powdering) the engineered CF-ECM. Lyophilization is a well-known technique used to prepare pharmaceutical compositions. In some cases, lyophilization is used to control fragment size.

As used herein, "pharmaceutical composition" means therapeutically effective amounts of the CF-ECM fragments together with suitable diluents, preservatives, solubilizers, emulsifiers, or adjuvants, collectively "pharmaceutically-acceptable carriers." As used herein, the terms "effective amount" and "therapeutically effective amount" refer to the quantity of active therapeutic agent sufficient to yield a desired therapeutic response without undue adverse side effects such as severe toxicity, severe irritation, or a severe allergic response. The specific "effective amount" will, obviously, vary with such factors as the particular condition being treated, the physical condition of the patient, the type of animal being treated, the duration of the treatment, the nature of concurrent therapy (if any), and the specific formulations employed and the structure of the compounds or its derivatives. The optimum effective amounts can be readily determined by one of ordinary skill in the art using routine experimentation.

Further, as used herein "pharmaceutically acceptable carriers" are well known to those skilled in the art and include, but are not limited to, 0.01-0.1M and preferably a 0.05M phosphate buffer or 0.9% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include but not limited to water, alcoholic/aqueous solutions, emulsions or suspensions, including saline and buffered media.

Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

The injectable composition may be for use in treating cardiac disease or injury, ischemic limb injury, or other injury due to the interruption of blood supply to a tissue. In some cases, the injectable composition is delivered into an endocardial wall of a heart chamber using any appropriate means for trans-endocardial delivery. For example, a delivery catheter can be used to deliver the injectable composition for treatment of a cardiac disease or condition. Other delivery devices can be used to achieve therapeutic or diagnostic delivery of an injectable composition as described herein. For example, the injectable composition can be delivered using a cardiac needle tip injection catheter such as the Myostar (Biosense Webster), Helix (Biocardia), Bullfrog (Mercator MedSystems) or C-Cath (Cardio3Biosciences). Advantageously, delivery of an injectable composition by the injection methods described herein is minimally invasive and can be achieved without general anesthesia, extracorporeal circulation (e.g., circulation via a heart-lung machine), circulatory support, or a chest opening. Accordingly, complication prospects and risks to the patient are substantially lower.

In some cases, the injectable composition is delivered to the outer heart wall (epicardium) using any appropriate means. for epicardial delivery. For example, epicardial delivery of an injectable composition described herein can be achieved using a delivery device comprising a needle and/or syringe.

The following Examples are offered for illustrative purposes only, and are not intended to limit the scope of the present invention in any way.

### EXAMPLES

### Example 1: Injectable Formulations of Engineered Cardiac Fibroblast Derived Extracellular Matrix

In this Example, we generated engineered ECM from cardiac fibroblasts, decellularized the engineered ECM, and fragmented the engineered ECM to make an injectable formulation of engineered CF-ECM. The resulting formulation was successfully delivered into a pig heart via catheter injection. Accordingly, this Example demonstrates an alternative composition of the engineered CF-ECM that can be delivered to the heart using minimally invasive non-surgical methods. Furthermore, the fragmented formulation greatly increases the surface area of the delivered engineered CF-ECM, resulting in enhanced benefits for both the previously disclosed cardiac disease or injury applications and in the ischemic wound applications first disclosed in this application.

### METHODS AND RESULTS

*Engineered Cardiac Fibroblast Derived Extracellular Matrix (CF-ECM).* Engineered fibroblast derived extracellular matrix was manufactured using methods that were previously described (see U.S. Patent No. 8,802,144 and Schmuck, E.G., et al., Cardiovasc Eng Technol 5(1) (2014): 119-131). Briefly, male Lewis rats (260-400g) were sacrificed by CO₂ asphyxiation, hearts rapidly excised, atria removed and ventricles placed into ice cold PBS with 1% penicillin/streptomycin. Hearts were finely minced and digested in Dulbecco's Modified Eagle's Medium (DMEM), 73 U/mL collagenase 2, 2 µg/mL pancreatin (4x) and incubated at 37°C with agitation for 65 min. The digested tissue was sieved through a 70 µm cell strainer and centrifuged at 1000xg for 20 min at 4°C. The cell pellet was re-suspended and plated into two T75 culture flasks. The cells were allowed to attach under standard culture conditions (37°C, 5% CO₂, 100% humidity) for 2 hours, then non-adherent cells removed by washing with PBS, and culture medium (DMEM, 10% Fetal Bovine Serum (FBS), 1% penicillin/streptomycin) replaced and cultured until confluent.

CF-ECM scaffold formation was induced by culturing cardiac fibroblasts (passage 2-3) plated at a density of approximately 1.1×10⁵ to 2.2×10⁵ per cm² in high glucose DMEM + 10% FBS and 1% penicillin/streptomycin and cultured at 37°C, 5% CO₂ and 100% humidity for 7 to 15 days. Fibroblasts were removed from the culture dish as a sheet by removing the medium and rinsing the fibroblasts with phosphate buffered saline (PBS). The cell sheets were then incubated with 2 mM EDTA in PBS solution at 37°C until they lifted off the plate surface (approximately five minutes).

*Optimized Engineered CF-ECM Decellularization Protocol.* The resulting cardiac fibroblast cell sheets were then denuded of cells. The sheets underwent two alternating 15 min. washes of Hypotonic /Hypertonic Tris buffered Saline (TBS) (Hypo, Hyper, Hypo, Hyper) at room temperature (RT) on a rotator. The Hypertonic TBS included 500mM NaCl/ 50mM Tris, and had a base pH of 7.6-8.0. The Hypotonic TBS was similarly prepared, except that it only included 10mM Tris (Base pH of 7.6-8.0).

Next, the sheets were washed for 72 hr. using1% Tri-n-Butyl Phosphate (TnBP) in Isotonic TBS at room temperature on a rotator. The Isotonic Tris included 150mM NaCl/ 50 mM Tris (Base pH 7.6-8.0). Subsequently, sheets were rinsed for 2 minutes in PBS, followed by a 3 hour wash in 90U/mL DNase (Qiagen) in Hypotonic TBS at 37°C on a rotator. This was followed by 2x2 minute rinses in Hypotonic TBS, after which the patches were moved to a different dish. This was followed by 2x2 minute washes in ethanol and a 2 hr. wash in Molecular Grade H₂O at room temperature on a rotator The resulting decellularized engineered CF-ECM were stored in PBS at 4°C until ready for use.

*Injectable CF-ECMFragmentation Protocol.* CF-ECM prepared as described above were diced with a razor blade, scalpel or scissors, until small (approximately 2 mm × 2 mm) fragments were produced. The small CF-ECM fragments were suspended in 1-5 mL isotonic buffer (saline, PBS, TBS, Plasmalyte A) and transferred to an appropriate sized tube. This suspension containing the CF-ECM fragments was then passed through an 18-gauge needle attached to a 3-5 ml syringe 15-20 times. The resulting CF-ECM suspension was subsequently passed through a 21-gauge needle attached to a 3-5 m syringe 15-20 times. The resulting CF-ECM suspension was passed through a 24 gauge needle attached to a 3-5 m syringe 15-20 times. The resulting CF-ECM suspension was passed through a 27 gauge needle attached to a 3-5 m syringe 15-20 times. The resulting CF-ECM suspension was then centrifuged at 5000-10,000 × g for 10-15 minutes.

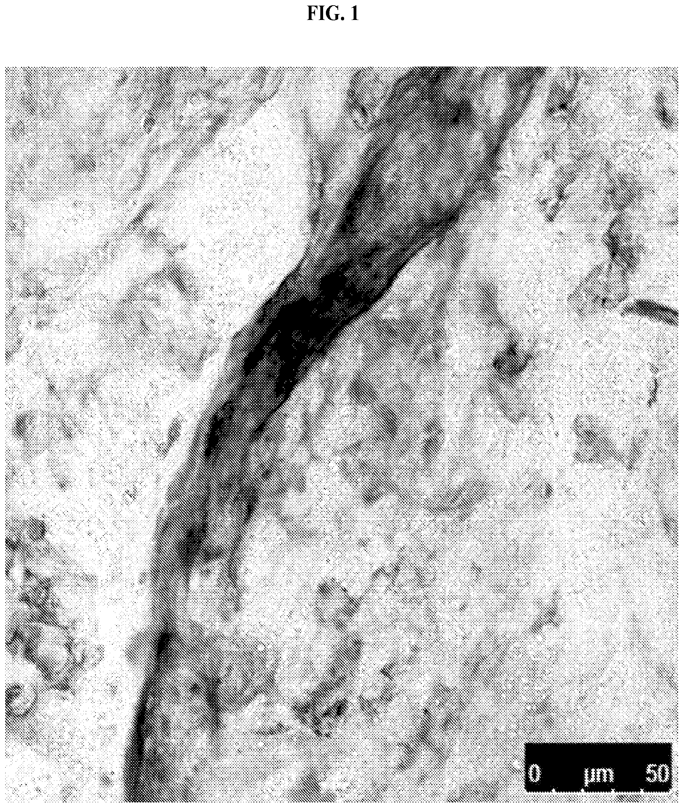

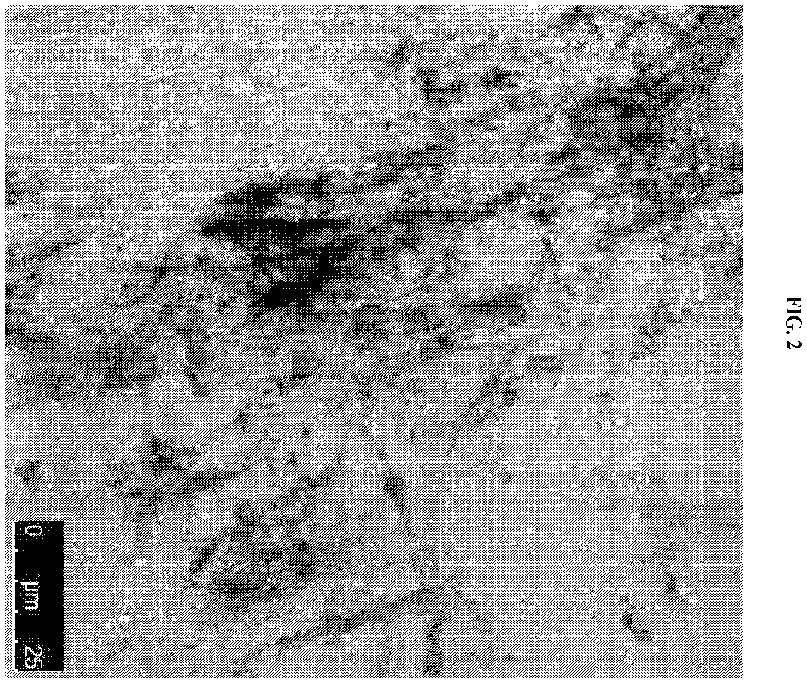

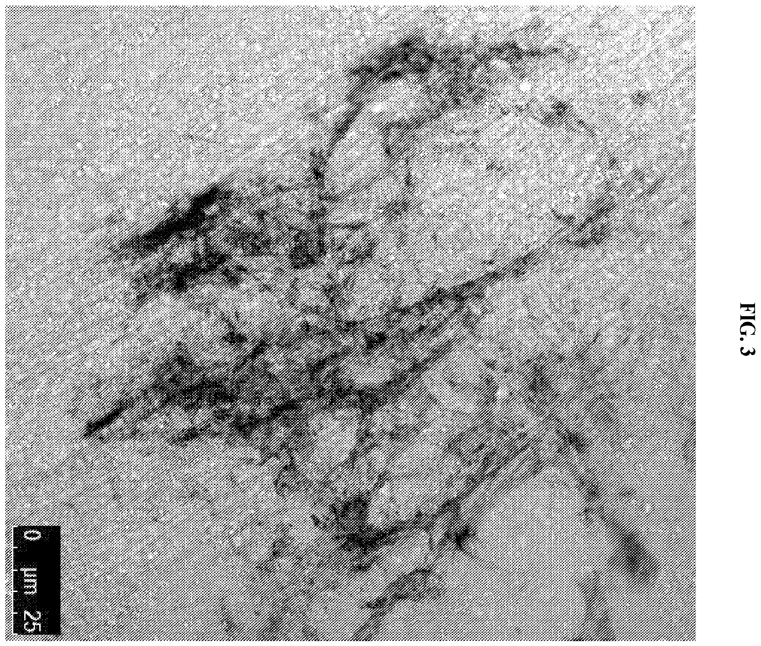

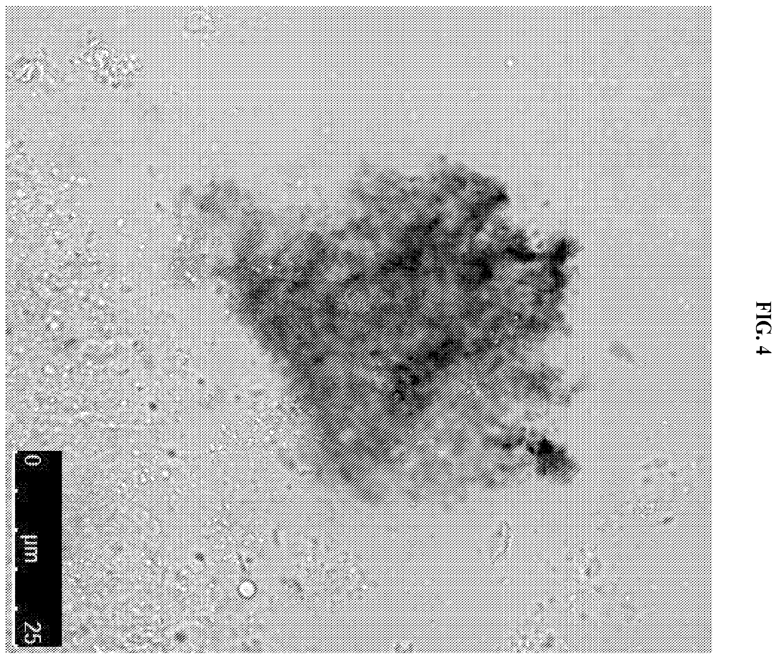

To form the final injectable composition, the fragmented CF-ECM was suspended of isotonic injectable solution (Normal saline, Plasmalyte A) in an appropriate volume for injection (0.5-5 mL). The fragmented CF-ECM are small, heterogeneous sheet-like fragments (see FIGS. 1-4).

### Testing of Injectable Fragmented CF-ECM Composition.

The injectable composition of fragmented CF-ECM was tested in a cardiac injection catheter (27 gauge needle tipped catheter). Tests confirm that the CF-ECM fragments can pass through the catheter without clogging despite a dwell time (time sitting in the catheter) of one hour. Further testing showed that the CF-ECM fragments bind readily to cells and can still function as seeded material. In one test, cells were bound to an intact CF-ECM scaffold then fragmented for injection. In another test, CF-ECM scaffolds were fragmented then cells bound to the fragment mixture.

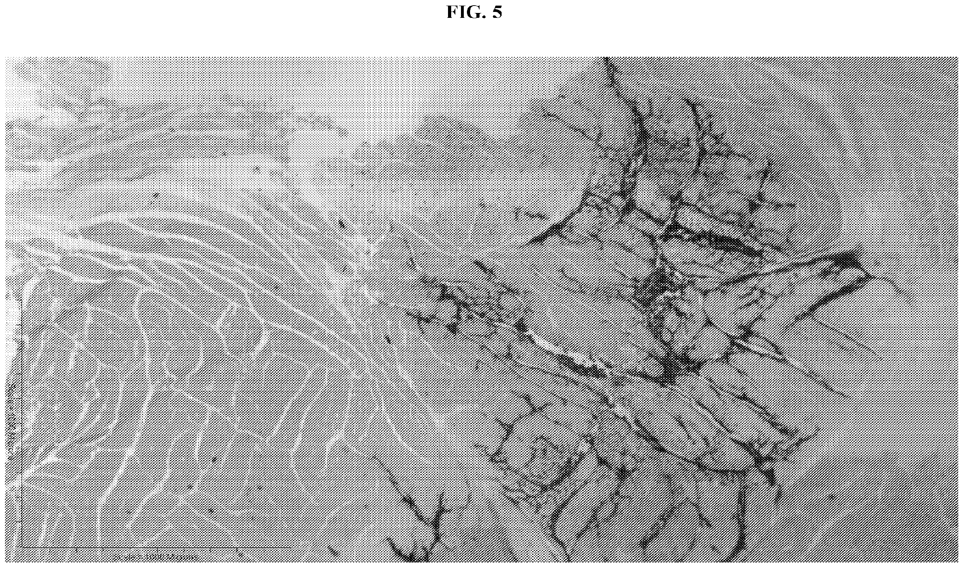

An injectable composition containing the CF-ECM fragments was injected into a pig ventricle via injection catheter. Further testing showed that the CF-ECM fragments were successfully delivered into the pig heart. Injectable composition of CF-ECM was detected in the ventricle by dyeing the CF-ECM black prior to injection. Injectable CF-ECM was primarily observed in the interstitial space of the left ventricle (see FIG. 5).

### CONCLUSION

This Example demonstrates that our injectable formulation containing fragmented engineered CF-ECM can be administered to the heart minimally invasively through injection cardiac catheters, such as the Helix or Myostar. In contrast, the previously disclosed CF-ECM "patch" must be implanted onto the target tissue by performing an invasive surgical procedure. Furthermore, by fragmenting the CF-ECM, the total surface area of the CF-ECM used is increased by over 40 times, potentially resulting in increased therapeutic efficacy.

As shown in the following examples, intact CF-ECM scaffolds can be used as a therapeutic agent in treating damaged cardiac tissue or ischemic limb injury, even in the absence of therapeutic cells. Thus, injectable CF-ECM fragment formulations can be used as a stand-alone therapy, or can be seeded with therapeutic cells for transplantation. In use as a therapeutic cell delivery platform, the CF-ECM fragments can be paired with any adherent cell type. Adherent cells rapidly attach to CF-ECM and can be delivered to the diseased myocardium (or any organ/tissue) by needle and syringe or needle tipped catheter, increasing cell retention in the targeted tissue.

### Example 2: Engineered CF-ECM "Patch" Shows Therapeutic Effects in a Rat Myocardial Infarction Model, Even in the Absence of Seeded Cells

Engineered CF-ECM were previously disclosed as a platform for delivering therapeutic cells to damaged or diseases cardiac tissue (see, e.g., U.S. Patent No. 8,802,144; Schmuck, E.G., et al., Cardiovasc Eng Technol 5(1) (2014): 119-131). In this Example, we report the surprising finding that the engineered CF-ECM "patch" has therapeutic effects even in the absence of therapeutic cells, as demonstrated in a rat model of myocardial infarction (MI).

### METHODS AND RESULTS

*Engineered Cardiac Fibroblast Derived Extracellular Matrix (CF-ECM).* Engineered fibroblast derived extracellular matrix was manufactured using methods that were previously described (see U.S. Patent No. 8,802,144 and Schmuck, E.G., et al., Cardiovasc Eng Technol 5(1) (2014): 119-131). Briefly, male Lewis rats (260-400g) were sacrificed by CO₂ asphyxiation, hearts rapidly excised, atria removed and ventricles placed into ice cold PBS with 1% penicillin/streptomycin. Hearts were finely minced and digested in Dulbecco's Modified Eagle's Medium (DMEM), 73 U/mL collagenase 2, 2 µg/mL pancreatin (4x) and incubated at 37°C with agitation for 65 min. The digested tissue was sieved through a 70 µm cell strainer and centrifuged at 1000xg for 20 min at 4°C. The cell pellet was re-suspended and plated into two T75 culture flasks. The cells were allowed to attach under standard culture conditions (37°C, 5% CO₂, 100% humidity) for 2 hours, then non-adherent cells removed by washing with PBS, and culture medium (DMEM, 10% Fetal Bovine Serum (FBS), 1% penicillin/streptomycin) replaced and cultured until confluent.

*Rat LAD permanent ligation model.* Myocardial infarction was induced in 12 week old male Lewis rats by left anterior descending artery ligation (LAD permanent ligation model; see Kumar D. et al., Coron Artery Dis. 2005 Feb;16(1):41-44). The LAD was ligated and the chest closed, modeling myocardial infarction (MI). 48 hours after MI, the chest was opened, and one of four different treatments performed: (1) a CF-ECM patch devoid of cells was placed onto the area of infarction (patch only); (2) a CF-ECM patch seeded for two hours with rat mesenchymal stem cells (MSCs) was placed on the area of infarction (seeded patch); (3) rat mesenchymal stem cells (MSCs) were placed on the area of infarction without a CF-ECM patch (cells only); and (4) no cells or CF-ECM patch was placed on the area of infarction (sham).

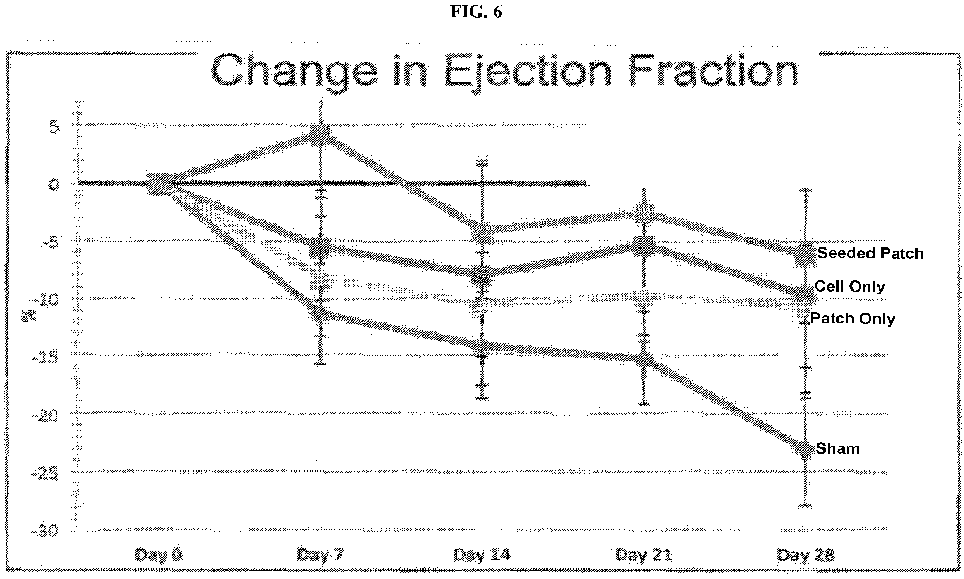

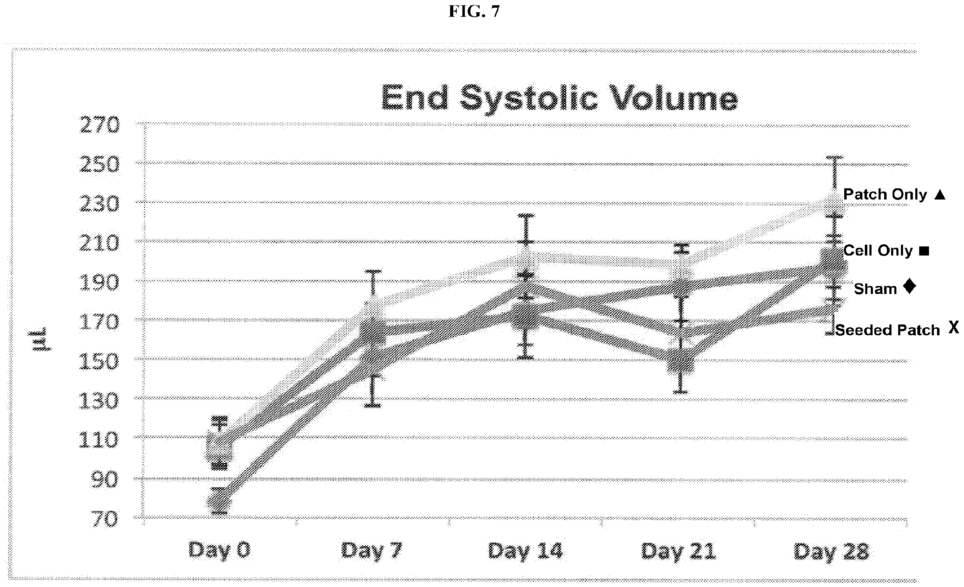

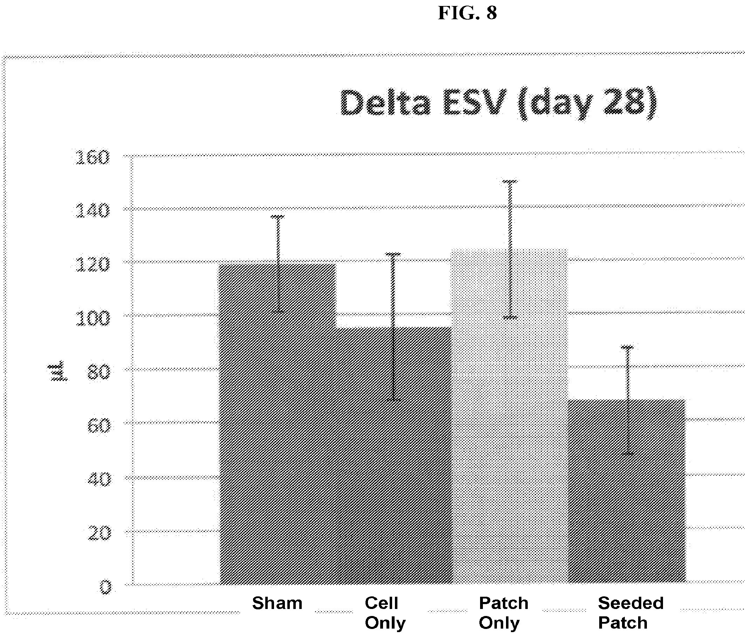

*Echocardiogram.* Serial echocardiograms were performed on each rat at days 0, 7, 14, 21, and 28 post MI. MI causes progressive deleterious left ventricle dilation. As measured by change in ejection fraction (FIG. 6) and end systolic volume (FIGS. 7 and 8), both the seeded patch and the patch produced a measurable therapeutic effect, as compared to the sham procedure.

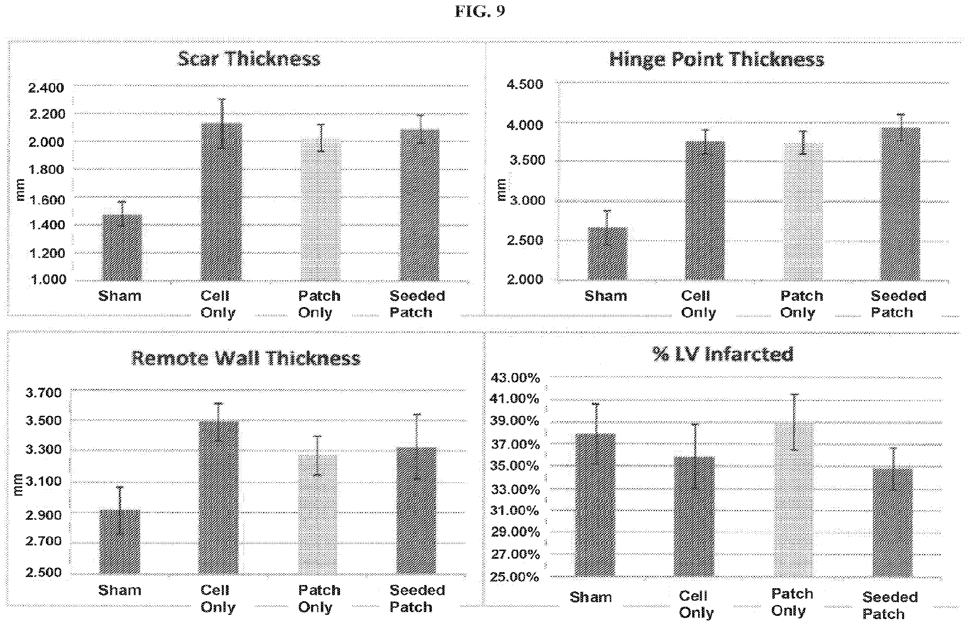

*Cross section pathology.* The rats were sacrificed, and cross section pathology was performed on the infarcted hearts. Specifically, scar thickness, hinge point thickness, remote wall thickness, and % left ventricle tissue infarcted were measured. Treatment with both the seeded patch and with the patch only resulted in increased hinge point thickness, scar thickness, and remote wall thickness, as compared to the sham treatment (FIG. 9).

### CONCLUSION

Together, these results surprisingly indicate that the cell free CF-ECM patch can used in the absence of seeded cells as a therapeutic agent for the treatment of cardiac disease or injury.

### Example 3: Engineered CF-ECM "Patch" Shows Therapeutic Effects in a Mouse Limb Ischemia Model, Both in the Presence or Absence of Seeded Cells

Engineered CF-ECM were previously disclosed as a platform for delivering therapeutic cells to damaged or diseases cardiac tissue (see, e.g., U.S. Patent No. 8,802,144; Schmuck, E.G., et al., Cardiovasc Eng Technol 5(1) (2014): 119-131). In this Example, we report the surprising finding that the engineered CF-ECM "patch" has therapeutic effects in a model of ischemic limb injury, both with or without therapeutic cells seeded onto the patch.

### METHODS

*Fibroblast derived extracellular matrix.* Engineered cardiac fibroblast derived extracellular matrix (CF-ECM) was manufactured using methods that were previously described (see U.S. Patent No. 8,802,144 and Schmuck, E.G., et al., Cardiovasc Eng Technol 5(1) (2014): 119-131). Briefly, male Lewis rats (260-400g) were sacrificed by CO₂ asphyxiation, hearts rapidly excised, atria removed and ventricles placed into ice cold PBS with 1% penicillin/streptomycin. Hearts were finely minced and digested in Dulbecco's Modified Eagle's Medium (DMEM), 73 U/mL collagenase 2, 2 µg/mL pancreatin (4x) and incubated at 37°C with agitation for 65 min. The digested tissue was sieved through a 70 µm cell strainer and centrifuged at 1000xg for 20 min at 4°C. The cell pellet was re-suspended and plated into two T75 culture flasks. The cells were allowed to attach under standard culture conditions (37°C, 5% CO₂, 100% humidity) for 2 hours, then non-adherent cells removed by washing with PBS, and culture medium (DMEM, 10% Fetal Bovine Serum (FBS), 1% penicillin/streptomycin) replaced and cultured until confluent.

CF-ECM scaffold formation was induced by culturing cardiac fibroblasts (passage 2-3) plated at a density of approximately 1.1×10⁵ to 2.2×10⁵ per cm² in high glucose DMEM + 10% FBS and 1% penicillin/streptomycin and cultured at 37°C, 5% CO₂ and 100% humidity for 10 to 14 days. Fibroblasts were removed from the culture dish as a sheet by incubation with 2 mM EDTA solution at 37°C. The resulting fibroblast cell sheet was then denuded of cells by incubation with molecular grade water followed by incubation with 0.15% peracetic acid for 24-48 hours at 4°C with constant agitation. The resulting matrix was then rinsed repeatedly with sterile water followed by PBS.

The resulting CF-ECM scaffold is approximately a 16mm diameter, 200 µm thick translucent scaffold that is easily handled and is naturally adherent to tissue, requiring no sutures or glue.

*GFP*+ *embryonic stem cell derived mesenchymal stem cells.* Human ESC lines H9 Cre-LoxP (constitutive EGFP expression) were obtained from WiCell (Madison, WI) at passage 22. Cells were cultured in mTeSR^{™}1 medium (StemCell Technologies) on Matrigel^{®} (BD Biosciences) coated flasks for 3-4 passages without removing differentiated areas. Differentiated cells were isolated and cultured in MSC growth medium (10% MSC characterized FBS, MEM non-essential amino acids, alpha-MEM) on tissue culture plastic until all cells had a fibroblast-like morphology. The cells exhibited the following flow cytometry profile: CD14-, CD31-, CD34-, CD45-, CD73+, CD90+ and CD105+. 7.5 × 10⁵ GFP⁺ MSC's were used seeded onto fibroblast extracellular matrix scaffolds for two hours prior to implantation or suspended in 500 µl of PBS for injection.

*In vitro* testing was performed on these cells to confirm MSC phenotype. For adipogenic and osteogenic differentiation, MSCs were plated in a 24-well plate and grown to confluency. Adipogenic and osteogenic differentiation media (Miltenyi Biotech, Auburn, CA) were added and changed every 3-4 days for a total of 21 days. Adipocyte lipid droplets were detected by oil red O staining (Sigma-Aldrich, St. Louis, MO). Osteoblast calcification was detected by alizarin red S staining (Sigma-Aldrich). For chondrogenic differentiation, 2.5×10⁵ cells were put in a deepwell 96-well plate and centrifuged to make pellets. Medium was changed every 3-4 days for a total of 24 days. Chondrocytes were detected using paraffin-embedded sections of the chondrocyte pellets stained with Alcian blue, which stains glucosaminoglycans.

*Mouse hind limb ischemia model and therapeutic delivery.* All procedures were carried out in accordance with the policies and guidelines of the UW-Madison institutional animal care and use committee. Hind limb ischemia model was created as previously described (Westvik TS, Fitzgerald TN, Muto A, Maloney SP, Pimiento JM, Fancher TT, et al. Limb ischemia after iliac ligation in aged mice stimulates angiogenesis without arteriogenesis. Journal of vascular surgery. 2009;49:464-473). Briefly, immune-competent female Balb/C mice weighing 18.1+/-1.4 g were anesthetized with 2-5% inhaled iso-fluorane. Animals were denuded of hair from the level of the xyphoid to caudal to the knee, then placed in the supine position on a heated water blanket and maintained on inhaled 1-3% isofluorane. The common iliac artery was accessed by midline incision. Using blunt dissection, the left common iliac artery was exposed, separated from the vein and surrounding tissue then double ligated with 6-0 silk approximately three mm apart. Following ligation the abdominal wall and skin were closed. The left femoral artery was accessed by inguinal incision. The femoral artery was isolated as described above and exposed distal to the inguinal ligament to proximal to the popliteal artery. Finally, the femoral artery was double ligated with 6-0 silk and bisected between ligations. Study agent was then applied either subcutaneously or intramuscularly (see below), the skin was closed, and animals were recovered.

The mice were divided into 4 treatment groups: Group A: CF-ECM loaded with 1.0 × 10⁶ GFP+ MSC's delivered sub-dermally onto the adductor muscles. Group B: CF-ECM only without cells delivered sub-dermally on to the adductor muscles. Group C: GFP⁺ MSC only delivered by intra-muscular injection into the adductor muscle. Group D: Control group consisted of saline delivered by intra-muscular injection into the adductor muscle. Intramuscular injections were performed using a 27G needle and a 1 mL syringe, injected approximately at 4 sites distributed around the femoral ligation into the abductor muscle. CF-ECM were oriented cell-side down and spread evenly to cover the femoral ligation.

*Endpoints.* Animals in all four groups were survived to 35 days following treatment and then sacrificed. Tissue necrosis and hind-limb perfusion were measured using digital photography, longitudinal laser Doppler assay, and semi-quantitative histopathology. In addition, a modified ischemia (MII) index score, as previously defined (see Westvik TS, Fitzgerald TN, Muto A, Maloney SP, Pimiento JM, Fancher TT, et al. Limb ischemia after iliac ligation in aged mice stimulates angiogenesis without arteriogenesis. Journal of vascular surgery. 2009;49:464-473), was calculated.

*Scanning Laser Doppler Perfusion.* Laser Doppler scanning was carried out on postoperative day 2, 7, 14, 21, 28, 35 using a moorLDI Laser Doppler Imager by Moor Instruments Ltd, Millwey Axminster, Devon, England. Briefly, mice were anesthetized with 2-5% inhaled isofluorane and maintained at 1.5% isoflurane for the duration of the scan. Mice were immobilized in the supine position with the ventral surface of the lower extremities contained within the Doppler scanner field (6.0 cm × 3.2 cm). Mice were scanned at a resolution of 10 ms/pixel. Room temperature was 24.4±1 °C. Three scans per animal per time point were completed and averaged for the final measurement. Analysis was carried out using moorLDI image software. Analysis was carried out at the center of the ventral surface of the paw just distal to the first digit using a 2 mm circular region of interest.

*Histopathology.* Hind limbs were isolated by disarticulation of the pelvis from the spinal column. The skin of the caudal limbs was then incised down the medial aspect to allow for proper fixation. The tissues were decalcified for 24 hours in Surgipath Decalcifier I (Leica) then fixed in 10% formalin. Tissues were cross-sectioned at the thigh and the calf, embedded in paraffin and 10 µm sections cut and stained with hemotoxylin and eosin. A quantitative scoring system was developed by the veterinary pathologist to assess nuclear centralization, fatty infiltration, bone marrow necrosis, fibrosis and myofiber heterogeneity.

### RESULTS

*GFP*+ *ESC derivedMSC delivered on CF-ECM results in dramatic improvement in perfusion in a mouse model of severe limb ischemia.* Severe hind limb ischemia was successfully induced by illeo-femoral ligation in 26 Balb/C mice. At the time of model creation mice were randomized into one four groups; Placebo injection, hMSC injection, CF-ECM scaffold only and hMSC seeded CF-ECM scaffolds. All mice survived surgery and treatment, two mice in the cell only injection group were excluded from analysis due to poor cell viability.

Severe ischemia was confirmed at post-operative day 2 by laser Doppler scanning (FIG. 10A). Over the course of the 35-day experiment there was a significant time treatment interaction (p=0.03) for perfusion in the CF-ECM only and hMSC seeded CF-ECM compared to placebo injection and hMSC injection (FIGS. 10A-10B). Animals treated with hMSC seeded CF-ECM had the lowest rate of auto-amputation (FIG. 10C). Modified Ischemia Index scores were greatest in the CF-ECM only and hMSC seeded CF-ECM groups compared to placebo injection and hMSC injection (FIG. 10D).

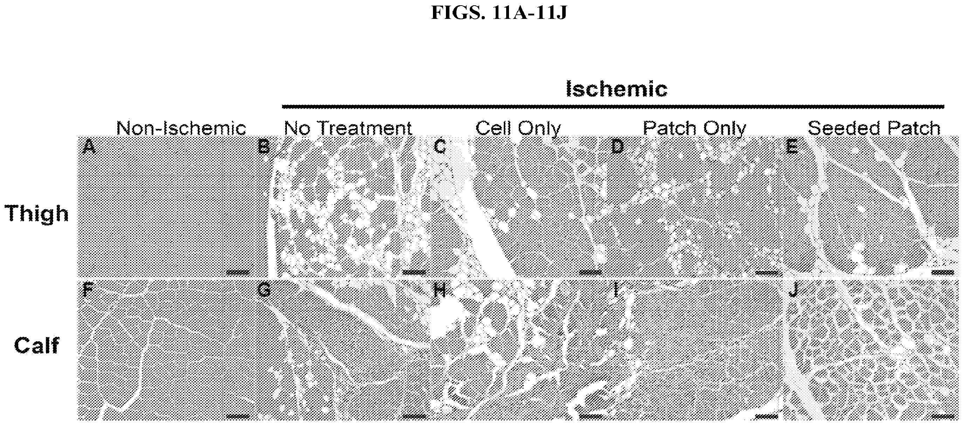

*Quantitative Histological Analysis.* Quantitative histological scoring of the ischemic calf (IC) and thigh (IT) was carried out by a certified veterinary pathologist (DS) (Table 1). Bone marrow necrosis, an indicator of severe ischemia, was marked to severe in all groups. Myofiber heterogeneity, a marker of regeneration, was significantly increased in the CF-ECM (IC = 3.7 +/-0.3; IT =3.0 +/-0.2) and hMSC seeded CF-ECM (IC = 3.8 +/-0.3, IT =3.2 +/-0.2) groups compared to placebo injection (IC = 2. +/-0.3, IT =2.1 +/-0.2) and hMSC injection (IC = 3.0 +/-0.4, IT =2.6 +/-0.2) groups only (p=0.04). There was no overall difference in nuclear centralization (IC p=0.35, IT p=0.59) fatty infiltration (IC p=0.37, IT p=0.63), bone marrow necrosis (IC p=0.32, IT p=0.64) and fibrosis (IC p=0.62, IT p=0.64). Myocytes per high-powered field in the IC (p=0.60) or IT (p=0.97) (FIG. 11) was unaffected by treatment but myocyte area was significantly reduced in all treatments in the IC (p=0.0001) and IT (0.0004).

Table 1 shows quantitative histological scoring of affected tissues. Note significant increase in myocyte heterogeneity in the CF-ECM treated animals compared to cell treated and sham controls in both the thigh (p=0.0035) and calf (p=0.05). Myocyte heterogeneity is a marker of muscle regeneration.

### CONCLUSION

This example shows that the engineered CF-ECM "patch" can used to effectively treat limb ischemia. The patch may be used as either a cell-free therapy, or seeded with potentially therapeutic cells.

**Table 1: Quantitative histological scoring of affected tissues**

| | **Nuclear Centralization** | | | | **Fatty Infiltration** | | | | |
|---|---|---|---|---|---|---|---|---|---|
| | Left Thigh | Left Calf | Right Thigh | Right Calf | Left Thigh | Left Calf | Right Thigh | Right Calf | |
| Control | 2.9+/-0.26 | 3.5+/-0.22 | 0.0+/-0.00 | 0.0+/-0.00 | 2.1+/-0.26 | 2.3+/-0.21 | 0.0+/-0.00 | 0.0+/-0.00 | |
| Cell only | 2.4+/-0.20 | 4.0+/-0.00 | 0.0+/-0.00 | 0.1+/-0.14 | 2.0+/-0.31 | 2.0+/-0.45 | 0.0+/-0.00 | 0.0+/-0.00 | |
| Patch Only | 2.3+/-0.42 | 4.0+/-0.00 | 0.0+/-0.00 | 0.0+/-0.00 | 1.8+/-0.40 | 2.2+/-0.31 | 0.0+/-0.00 | 0.0+/-0.00 | |
| Seeded Patch | 2.5+/-0.22 | 3.7+/-0.33 | 0.0+/-0.00 | 0.2+/-0.17 | 2.0+/-0.26 | 2.0+/-0.45 | 0.0+/-0.00 | 0.0+/-0.00 | |

| | **Bone Marrow Necrosis** | | | | **Fibrosis** | | | | |
|---|---|---|---|---|---|---|---|---|---|
| | Left Thigh | Left Calf | Right Thigh | Right Calf | Left Thigh | Left Calf | Right Thigh | Right Calf | |
| Control | 0.6+/-0.57 | 3.4+/-0.20 | 0.0+/-0.00 | 0.0+/-0.00 | 0.3+/-0.29 | 1.0+/-0.45 | 0.0+/-0.00 | 0.0+/-0.00 | |
| Cell only | 0.6+/-0.57 | 3.9+/-0.14 | 0.0+/-0.00 | 0.1+/-0.14 | 0.0+/-0.00 | 1.4+/-0.51 | 0.0+/-0.00 | 0.0+/-0.00 | |
| Patch Only | 0.0+/-0.00 | 3.8+/-0.17 | 0.0+/-0.00 | 0.0+/-0.00 | 0.5+/-0.34 | 1.5+/-0.43 | 0.0+/-0.00 | 0.0+/-0.00 | |
| Seeded Patch | 1.0+/-0.63 | 3.8+/-0.17 | 0.0+/-0.00 | 0.2+/-0.17 | 0.5+/-0.34 | 1.8+/-0.60 | 0.0+/-0.00 | 0.0+/-0.00 | |

| | | | | | **Grading System** | | | | |
|---|---|---|---|---|---|---|---|---|---|
| | **Myofiber Heterogeneity** | | | | **Nuclear Centralization, Fatty Infiltration and Bone Marrow Necrosis** | | | **Fibrosis** | **Myofiber Heterogeneity** |
| | Left Thigh | Left Calf | Right Thigh | Right Calf | | | | | |
| | | | | | **Grade 0** | 0-5% of cells | None | | Minimal |
| Control | 2.1+/-0.14 | 2.8+/-0.31 | 1.0+/-0.00 | 0.9+/-0.14 | **Grade 1** | 6-34% of cells | Focal | | Mild |
| Cell only | 2.4+/-0.20 | 3.2+/-0.37 | 1.0+/-0.00 | 1.0+/-0.22 | **Grade 2** | 35-66% of cells | Multifocal | | Moderate |
| Patch Only | 3.0+/-0.26+ | 3.7+/-0.21# | 1.0+/-0.00 | 1.0+/-0.00 | **Grade 3** | 67-95% of cells | Multifocal coalescing | | Marked |
| Seeded Patch | 3.2+/-0.17*^ | 3.8+/-0.17$ | 1.0+/-0.00 | 1.0+/-0.26 | **Grade 4** | 96-100% of cells | Diffuse | | Severe |
| Left Thigh MH are different by ANOVA p=.0035 | | | | | | | | | |
| t-test between groups: | | | | | | | | | |
| *p=.007 vs control | | | | | | | | | |
| ^p=0.02 vs cell only | | | | | | | | | |
| +p=0.01 vs control | | | | | | | | | |
| Left Calf MH are different by ANOVA p=.05 | | | | | | | | | |
| t-test between groups: | | | | | | | | | |
| $p=0.02 vs control | | | | | | | | | |
| #p=0.05 vs control | | | | | | | | | |

### Example 4: Engineered CF-ECM Sheet Shows Therapeutic Effects Even in the Absence of Seeded Cells

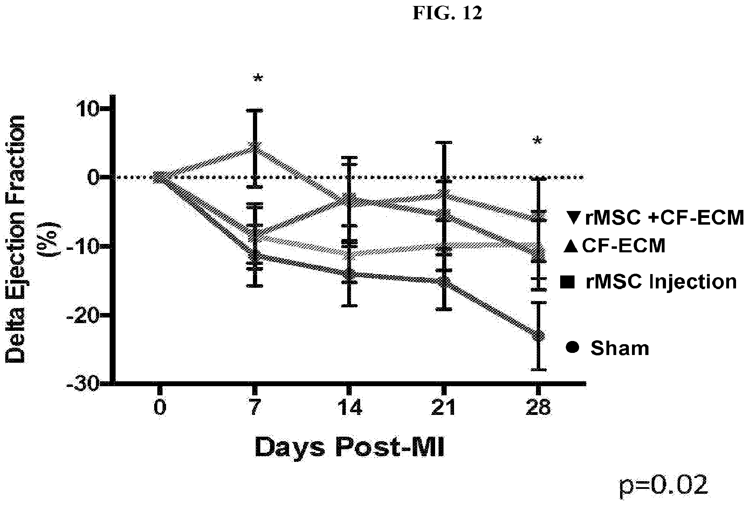

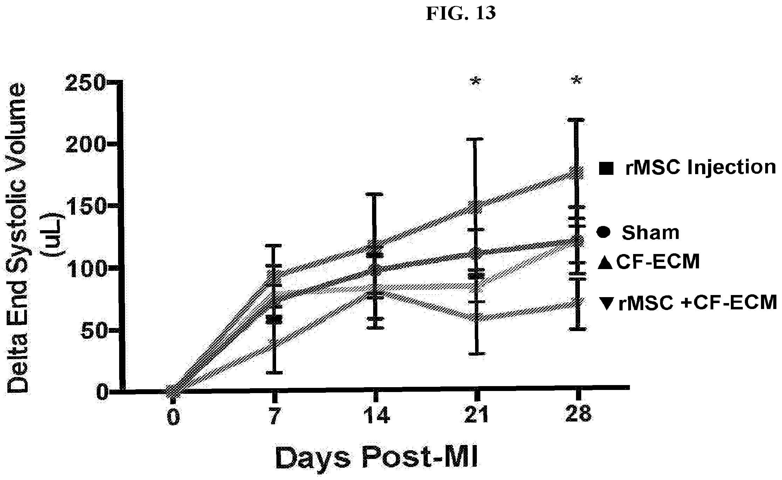

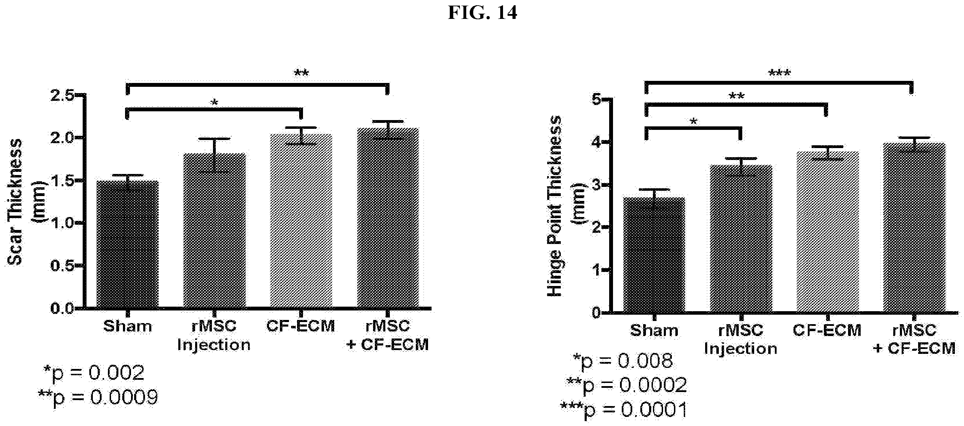

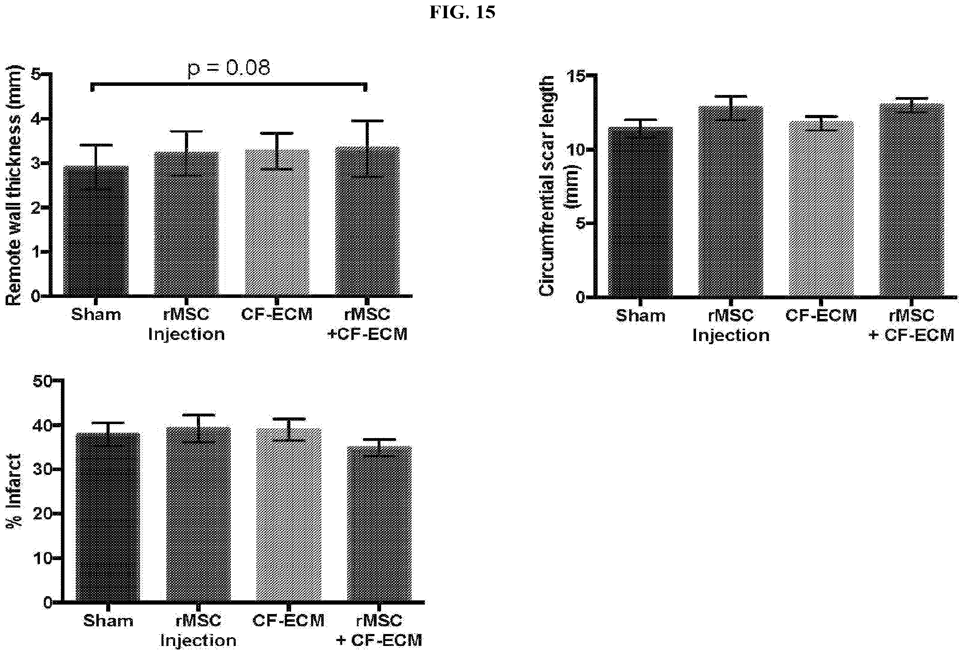

A rat myocardial infarction model was used to assay CF-ECM efficacy with and without seeded cells. When rat MSCs (rMSCs) were transferred with CF-ECM, post-MI delta ejection fractions were preserved (FIG. 12), end systolic volumes were increased (FIG. 13) and post-MI remodeling was decreased (FIGS. 14 and 15). The combination of CF-ECM + MSCs improved end systolic volumes, ejection fractions, hinge point thickness, and scar thickness. Even in the absence of seeded cells, CF-ECM alone improved ejection fractions, hinge point thickness, and scar thickness.

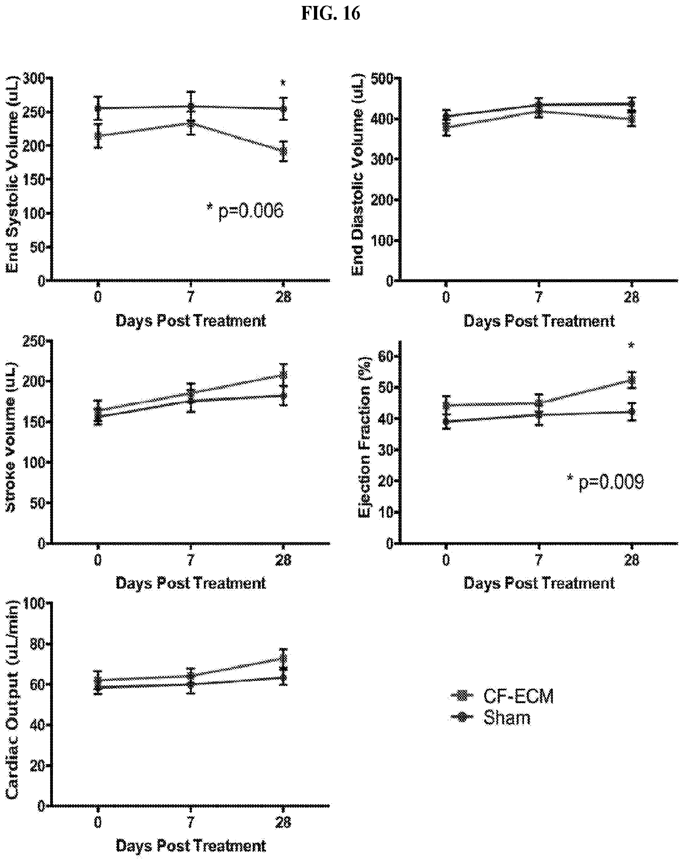

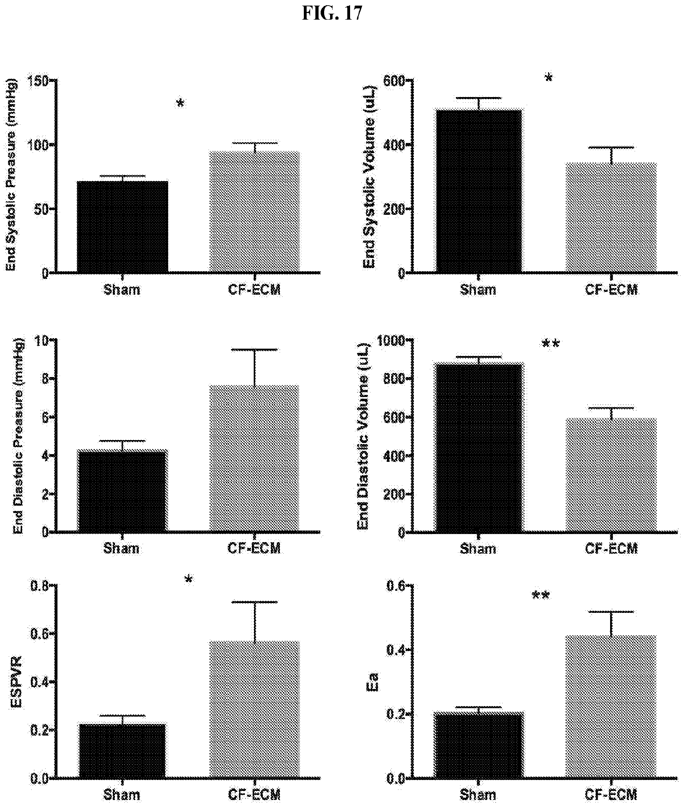

As shown in FIGS. 16 and 17, administration of CF-ECM alone was effective to reduce end systolic volumes (ESV) and to increase ejection fractions relative to sham treatment.

Together, these assays demonstrated that administration of CF-ECM alone was sufficient to improve ejection fractions, end systolic volumes, end systolic pressures, end diastolic volumes, and end systolic pressure volume relationship (ESPVR). Thus, administration of CF-ECM alone was beneficial.

### Example 5: Mesenchymal Stem Cell (MSC) Injection and Retention Assays

Injectable CF-ECM provides a number of advantages for clinical uses. For example, injection of CF-ECM is minimally invasive and can be delivered for cardiac regenerative therapies without a need for open heart surgery. This is important since many for whom cardiac cell therapies would be beneficial are too sick for open heart procedures. Minimally invasive injection of injectable CF-ECM would increase the patient pool that could receive cardiac cell therapies. Injectable CF-ECM can be provided to more areas of the heart, including direct delivery to the heart wall without requiring cell migration for beneficial placement of transplanted cells.

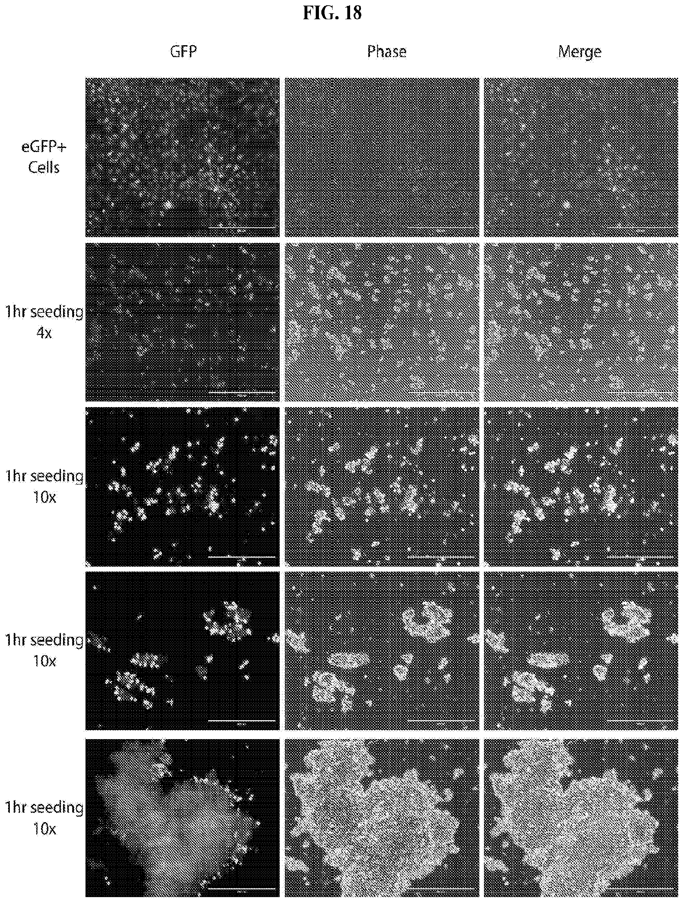

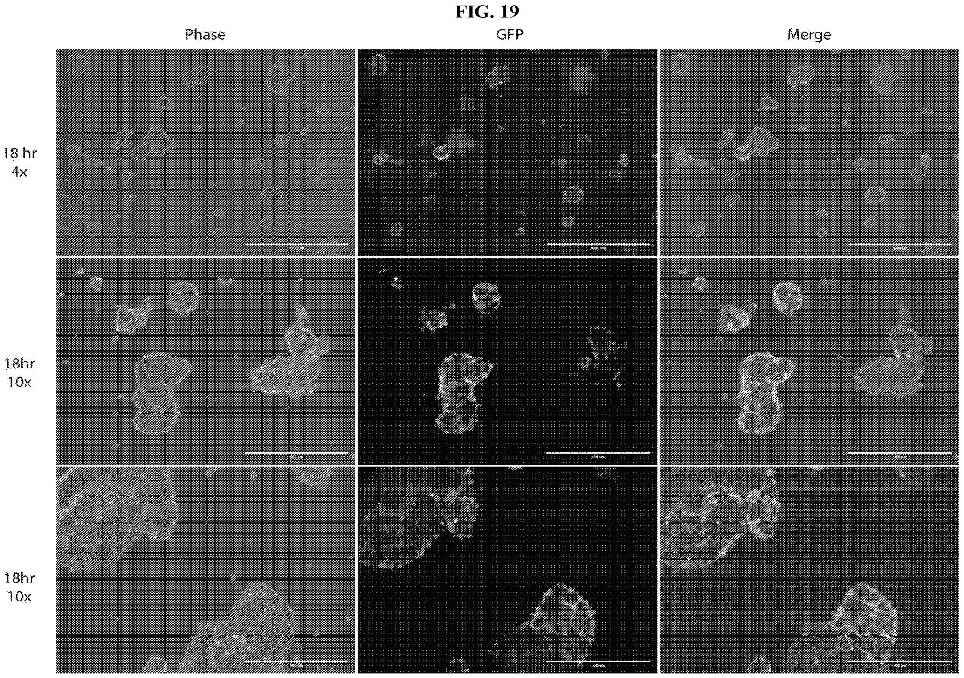

CF-ECM was seeded with GFP⁺ MSCs for 1 hour and 18 hours. As shown in FIGS. 18 and 19, respectively, MSCs migrated into aggregates of CF-ECM material.

Cell retention assays were performed using healthy rats (no myocardial infarction; n =5). Hearts were injected with: (1) 1 × 10⁶ rMSCs labeled with QTRACKER^{™} 525 probe; (2) 1 × 10⁶ rMSCs labeled with QTRACKER^{™} 655 probe and seeded into injectable CF-ECM. Data were collected at the following time-points: 4 hours, 24 hours, 48 hours, 6 days, and 7 days. Cell retention was evaluated using 3D cryo-imaging.

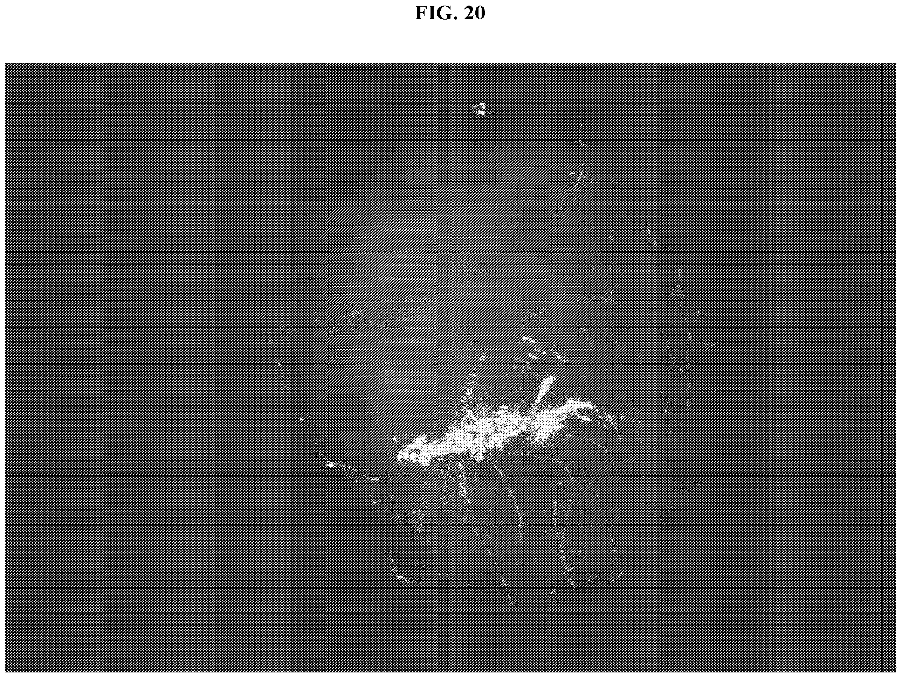

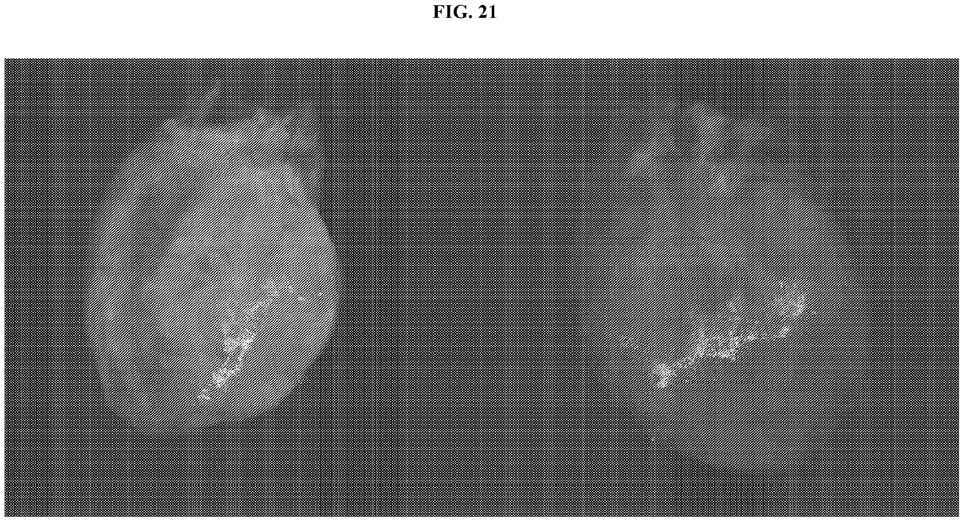

As shown in FIG. 20, injectable CF-ECM increased cell retention four (4) hour post-injection. As shown in FIG. 21, injectable CF-ECM increased cell retention 24- and 48-hours post-injection. Given the similar distribution to rMSCs bound to CF-ECM that was observed in the 4 hour time point, it is likely that QTRACKER^{™} 655 probe was lost and the redundant GFP signal is being misinterpreted as "naked" rMSC.

These data demonstrate that an injectable formulation of CF-ECM can be delivered using a needle tip cardiac catheter, and that CF-ECM dramatically increased cell retention in the heart wall at 4 hours compared to "naked" injection of MSCs (i.e., injected without injectable CF-ECM). Utilization of a cell line expressing eGFP made interpretation of data difficult after the 4 hour time point. For instance, QTRACKER^{™} 655 probe reporter expression was difficult to detect after 24 hours.

In summary, CF-ECM can be manufactured as a sheet or injectable formulation. Both formulations allow for targeted delivery of cells. Therapeutic cell retention was greatly improved using both sheet and injectable formulations. In addition, we demonstrated that CF-ECM has innate bioactivity on its own.

## Claims

1. An injectable composition for treating cardiac disease, cardiac injury, or ischemic injury, comprising:
(a) fragments of an engineered cardiac fibroblast cell-derived extracellular matrix (CF-ECM) comprising structural proteins fibronectin, collagen type I, collagen type III, and elastin, wherein from 60% to 90% of the structural proteins present in the engineered extracellular matrix are fibronectin, wherein the one or more fragments are small enough to pass through the injection opening of an 18-gauge hypodermic needle; and
(b) an injectable pharmaceutically acceptable carrier, wherein the fragments are suspended in the carrier.

2. The composition of claim 1, wherein the structural proteins of the engineered cardiac extracellular matrix are not chemically cross-linked.

3. The composition of claim 1 or 2, wherein the one or more fragments have a thickness of 20-500 µm.

4. The composition of any one of claims 1-3, wherein the one or more fragments are in the form of a lyophilized powder.

5. The composition of any one of claims 1-4, wherein the engineered cardiac extracellular matrix further comprises one or more of the matricellular proteins latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, and biglycan.

6. The composition of any one of claims 1-5, further comprising one or more exogenous non-cellular therapeutic agents, optionally wherein the one or more exogenous non-cellular therapeutic agents include one or more growth factors, optionally wherein the one or more growth factors are selected from the group consisting of epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), stromal derived factor-1 (SDF-1), and keratinocyte growth factor (KGF).

7. The composition of any one of claims 1-6, wherein: (a) the composition is devoid of intact cardiac fibroblast cells; or (b) the composition is cell free.

8. The composition of any one of claims 1-6, further comprising one or more cells that are therapeutic for cardiac disease, cardiac injury, or ischemic injury, optionally wherein the one or more cells that are therapeutic for cardiac disease, cardiac injury, or ischemic injury are selected from the group consisting of skeletal myoblasts, embryonic stem (ES) cells or derivatives thereof, induced pluripotent stem (iPS) cells or derivatives thereof, multipotent adult germline stem cells (maGCSs), bone marrow mesenchymal stem cells (BMSCs), very small embryonic-like stem cells (VSEL cells), endothelial progenitor cells (EPCs), cardiopoietic cells (CPCs), cardiosphere-derived cells (CDCs), multipotent *Isl1*+ cardiovascular progenitor cells (MICPs), epicardium-derived progenitor cells (EPDCs), adipose-derived stem cells, human mesenchymal stem cells derived from iPS or ES cells, human mesenchymal stromal cells derived from iPS or ES cells, and combinations thereof.

9. The composition of any one of claims 1-8, wherein the fragments are small enough to pass through the injection opening of a 27-gauge hypodermic needle.

10. The composition of any one of claims 1-9 for use in a method for treating a subject having a cardiac disease, cardiac injury, ischemic disease or ischemic injury, the method comprising injecting into the subject an effective amount of the composition, whereby the severity of the cardiac disease, cardiac injury, ischemic disease or ischemic injury is decreased.

11. The composition for use according to claim 10, wherein the cardiac disease, cardiac injury, ischemic disease or ischemic injury is caused by an acute myocardial infarct, heart failure, viral infection, bacterial infection, congenital defect, stroke, diabetic foot ulcer, peripheral artery disease (PAD), PAD-associated ulcer, or limb ischemia.

12. A cell free engineered cardiac fibroblast cell-derived extracellular matrix for use in a method for treating a subject having an ischemic disease or injury, the method comprising injecting the injectable composition of any one of claims 1 to 6 into a tissue of the subject that is exhibiting an ischemic disease or injury, wherein no cells are seeded onto the engineered cardiac fibroblast cell-derived extracellular matrix,
whereby the severity of the ischemic disease or injury is decreased.

13. The matrix for use according to claim 12, wherein the cell free engineered cardiac extracellular matrix further comprises one or more of the matricellular proteins latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, and biglycan.

14. The matrix for use according to claim 12 or 13, wherein the method further comprises contacting the tissue of the subject that is exhibiting an ischemic disease or injury with one or more exogenous non-cellular therapeutic agents; optionally wherein the one or more non-cellular therapeutic agents include one or more growth factors, optionally wherein the one or more growth factors are selected from the group consisting of stromal derived factor-1 (SDF-1), epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), and keratinocyte growth factor (KGF).

15. The matrix for use according to any one of claims 12-14, wherein the ischemic disease or injury is caused by myocardial infarct, stroke, diabetic foot ulcer, peripheral artery disease (PAD), PAD-associated ulcer, or limb ischemia, optionally wherein the limb ischemia is the result of atherosclerosis or diabetes.

16. An engineered cardiac fibroblast cell-derived extracellular matrix for use in a method for treating a subject having an ischemic limb injury, the method comprising injecting into a tissue of the subject that is exhibiting an ischemic limb injury the fragments of an the engineered cardiac fibroblast cell-derived extracellular matrix, the fragments having a thickness of 20-500 µm comprising the structural proteins fibronectin, collagen type I, collagen type III, and elastin, wherein from 60% to 90% of the structural proteins present in the engineered extracellular matrix are fibronectin, wherein the engineered cardiac fibroblast cell-derived extracellular matrix is fragmented and suspended in an isotonic solution to form an injectable suspension and is then administered to the subject by injection,
whereby the severity of the ischemic limb injury is decreased.

17. The matrix for use according to claim 16, wherein the engineered cardiac extracellular matrix further comprises one or more of the matricellular proteins latent transforming growth factor beta 1 (LTGFB-1), latent transforming growth factor beta 2 (LTGFB-2), connective tissue growth factor (CTGF), secreted protein acidic and rich in cysteine (SPARC), versican core protein (VCAN), galectin 1, galectin 3, matrix gla protein (MGP), sulfated glycoprotein 1, and biglycan.

18. The matrix for use according to claim 16 or 17, wherein the engineered cardiac extracellular matrix is seeded with one or more cells that are therapeutic for ischemic limb injury, optionally wherein the one or more cells that are therapeutic for ischemic limb injury are selected from the group consisting of skeletal myoblasts, embryonic stem (ES) cells or derivatives thereof, induced pluripotent stem (iPS) cells or derivatives thereof, multipotent adult germline stem cells (maGCSs), bone marrow mesenchymal stem cells (BMSCs), very small embryonic-like stem cells (VSEL cells), endothelial progenitor cells (EPCs), adipose-derived stem cells, human mesenchymal stem cells derived from iPS or ES cells, human mesenchymal stromal cells derived from iPS or ES cells, and combinations thereof.

19. The matrix for use according to any one of claims 16-18, wherein the method further comprises contacting the tissue of the subject that is exhibiting an ischemic injury with one or more exogenous non-cellular therapeutic agents, optionally wherein the one or more non-cellular therapeutic agents include one or more growth factors, optionally wherein the one or more growth factors are selected from the group consisting of stromal derived factor-1 (SDF-1), epidermal growth factor (EDF), transforming growth factor-α (TGF-α), hepatocyte growth factor (HGF), vascular endothelial growth factor (VEGF), platelet derived growth factor (PDGF), fibroblast growth factor 1 (FGF-1), fibroblast growth factor 2 (FGF-2), transforming growth factor-β (TGF-β), and keratinocyte growth factor (KGF).

20. The matrix for use according to any one of claims 16-19, wherein the ischemic limb injury is the result of atherosclerosis.

## Patentansprüche

1. Injizierbare Zusammensetzung zur Behandlung von Herzerkrankungen, Herzverletzungen oder ischämischen Verletzungen, die umfasst:
(a) Fragmente einer künstlich hergestellten, von Herzfibroblastenzellen abgeleiteten extrazellulären Matrix (CF-ECM), die die Strukturproteine Fibronektin, Kollagen Typ I, Kollagen Typ III und Elastin umfasst, wobei 60 % bis 90 % der Strukturproteine, die in der manipulierten extrazellulären Matrix vorhanden sind, Fibronectin sind, wobei das eine oder die mehreren Fragmente klein genug sind, um durch die Injektionsöffnung einer 18-Gauge-Spritzennadel zu gelangen; und
(b) einen injizierbaren pharmazeutisch annehmbaren Träger, wobei die Fragmente in dem Träger suspendiert sind.

2. Zusammensetzung nach Anspruch 1, wobei die Strukturproteine der künstlich hergestellten extrazellulären Herzmatrix nicht chemisch vernetzt sind.

3. Zusammensetzung nach Anspruch 1 oder 2, wobei das eine oder die mehreren Fragmente eine Dicke von 20-500 µm aufweisen.

4. Zusammensetzung nach einem der Ansprüche 1-3, wobei das eine oder die mehreren Fragmente in Form eines gefriergetrockneten Pulvers vorliegen.

5. Zusammensetzung nach einem der Ansprüche 1-4, wobei die manipulierte extrazelluläre Herzmatrix ferner eines oder mehrere von Folgendem umfasst: latenten transformierenden Wachstumsfaktor beta 1 (LTGFB-1) der matrizellulären Proteine, latenten transformierenden Wachstumsfaktor beta 2 (LTGFB-2), Bindegewebswachstumsfaktor (Connective Tissue Growth Factor, CTGF) , saures und cysteinreiches sekretiertes Protein (Secreted Protein Acidic and Rich in Cysteine, SPARC), Versican-Kernprotein (VCAN), Galectin 1, Galectin 3, Matrix-Gla-Protein (MGP), sulfatiertes Glykoprotein 1 und Biglycan.

6. Zusammensetzung nach einem der Ansprüche 1-5, die ferner ein oder mehrere exogene nicht-zelluläre therapeutische Mittel umfasst, wobei optional der eine oder die mehreren exogenen nicht-zellulären therapeutischen Wirkstoffe einen oder mehrere Wachstumsfaktoren enthalten, wobei optional der eine oder die mehreren Wachstumsfaktoren ausgewählt sind aus der Gruppe bestehend aus epidermaler Wachstumsfaktor (EDF), transformierender Wachstumsfaktor-α (TGF-α), Hepatozyten-Wachstumsfaktor (HGF), vaskulärer endothelialer Wachstumsfaktor (VEGF), von Blutplättchen abgeleiteter Wachstumsfaktor (PDGF), Fibroblasten-Wachstumsfaktor 1 (FGF-1), Fibroblasten-Wachstumsfaktor 2 (FGF-2), transformierender Wachstumsfaktor-β (TGF-β), stromal abgeleiteter Faktor-1 (SDF-1) und Keratinozyten-Wachstumsfaktor (KGF).

7. Zusammensetzung nach einem der Ansprüche 1-6, wobei: (a) die Zusammensetzung frei von intakten Herzfibroblastenzellen ist; oder (b) die Zusammensetzung zellfrei ist.

8. Zusammensetzung nach einem der Ansprüche 1-6, die ferner eine oder mehrere Zellen umfasst, die für Herzerkrankungen, Herzverletzungen oder ischämische Verletzungen therapeutisch sind, wobei optional die eine oder mehrere Zellen, die für eine Herzerkrankung, eine Herzverletzung oder eine ischämische Verletzung therapeutisch sind, aus der Gruppe ausgewählt sind, die aus Folgendem besteht: Skelettmyoblasten, embryonalen Stammzellen (ES) oder Derivaten davon, induzierten pluripotenten Stammzellen (iPS) oder Derivaten davon, multipotenten adulten Keimbahnstammzellen (multipotent adult Germline Stem Cells, maGCSs), mesenchymalen Stammzellen aus dem Knochenmark (Bone Marrow Mesenchymal Stem Cells, BMSCs), sehr kleinen embryonalähnlichen Stammzellen (Very Small Embryonic Like, VSEL-Zellen), endothelialen Vorläuferzellen (Endothelial Vorläufer Cells, EPCs), kardiopoetischen Zellen (Cardiopoietic Cells, CPCs), aus der Kardiosphäre stammenden Zellen (Cardiosphere-Derived Cells, CDCs), multipotente Isl1 + kardiovaskulären Vorläuferzellen (Multipotent Isll + Cardiovascular Vorläufer Cells, MICPs), Epicardium-abgeleiteten Vorläuferzellen (EPDCs), adipose-abgeleiteten Stammzellen, humanen mesenchymalen Stammzellen, die von iPS- oder ES-Zellen abgeleitet sind, humanen mesenchymalen Stromazellen, die von iPS- oder ES-Zellen abgeleitet sind, und Kombinationen davon.

9. Zusammensetzung nach einem der Ansprüche 1-8, wobei die Fragmente klein genug sind, um durch die Injektionsöffnung einer 27-Gauge-Spritzennadel zu passen.

10. Zusammensetzung nach einem der Ansprüche 1-9 zur Verwendung in einem Verfahren zur Behandlung eines Subjekts mit einer Herzerkrankung, Herzverletzung, ischämischen Erkrankung oder ischämischen Verletzung, wobei das Verfahren die Injektion einer wirksamen Menge der Zusammensetzung in das Subjekt umfasst, wodurch der Schweregrad der Herzerkrankung, Herzverletzung, ischämischen Erkrankung oder ischämischen Verletzung verringert wird.

11. Zusammensetzung zur Verwendung nach Anspruch 10, wobei die Herzerkrankung, die Herzverletzung, die ischämische Erkrankung oder die ischämische Verletzung durch einen akuten Myokardinfarkt, eine Herzinsuffizienz, eine virale Infektion, eine bakterielle Infektion, einen angeborenen Defekt, einen Schlaganfall, ein diabetisches Fußgeschwür, eine periphere Arterienerkrankung (Peripheral Artery Disease, PAD), ein PAD-assoziiertes Geschwür oder eine Extremitätenischämie verursacht wird.

12. Aus Herzfibroblastenzellen gewonnene zellfreie extrazelluläre Matrix zur Verwendung in einem Verfahren zur Behandlung eines Subjekts mit einer ischämischen Erkrankung oder Verletzung, wobei das Verfahren die Injektion der injizierbaren Zusammensetzung nach einem der Ansprüche 1 bis 6 in ein Gewebe des Subjekts umfasst, das eine ischämische Erkrankung oder Verletzung aufweist, wobei keine Zellen auf die aus Herzfibroblastenzellen abgeleitete extrazelluläre Matrix ausgesät werden,
wodurch der Schweregrad der ischämischen Erkrankung oder Verletzung verringert wird.

13. Matrix zur Verwendung nach Anspruch 12, wobei die zellfrei hergestellte extrazelluläre Herzmatrix ferner eines oder mehrere von Folgendem umfasst: latenter transformierender Wachstumsfaktor beta 1 (LTGFB-1) der matrizellulären Proteine, latenter transformierender Wachstumsfaktor beta 2 (LTGFB-2), Bindegewebswachstumsfaktor (CTGF), saures und cysteinreiches sekretiertes Protein (SPARC), Versican-Kernprotein (VCAN), Galectin 1, Galectin 3, Matrix-Gla-Protein (MGP), sulfatiertes Glykoprotein 1 und Biglycan.

14. Matrix zur Verwendung nach Anspruch 12 oder 13, wobei das Verfahren ferner das Inkontaktbringen des Gewebes des Subjekts, das eine ischämische Erkrankung oder Verletzung aufweist, mit einem oder mehreren exogenen nicht-zellulären therapeutischen Mitteln umfasst; wobei optional das eine oder die mehreren nicht-zellulären therapeutischen Mittel einen oder mehrere Wachstumsfaktoren enthalten, wobei optional der eine oder die mehreren Wachstumsfaktoren ausgewählt sind aus der Gruppe bestehend aus stromal abgeleitetem Faktor-1 (SDF-1), epidermalem Wachstumsfaktor (EDF), transformierendem Wachstumsfaktor-α (TGF-α), Hepatozyten-Wachstumsfaktor (HGF), vaskulärem endothelialem Wachstumsfaktor (VEGF), von Plättchen abgeleitetem Wachstumsfaktor (PDGF), Fibroblasten-Wachstumsfaktor 1 (FGF-1), Fibroblasten-Wachstumsfaktor 2 (FGF-2), transformierendem Wachstumsfaktor-β (TGF-β) und Keratinozyten-Wachstumsfaktor (KGF).

15. Matrix zur Verwendung nach einem der Ansprüche 12-14, wobei die ischämische Erkrankung oder Verletzung durch einen Myokardinfarkt, einen Schlaganfall, ein diabetisches Fußgeschwür, eine periphere Arterienerkrankung (PAD), ein PAD-assoziiertes Geschwür oder eine Extremitätenischämie verursacht wird, optional wobei optional die Ischämie der Gliedmaßen das Ergebnis von Atherosklerose oder Diabetes ist.

16. Aus Herzfibroblastenzellen abgeleitete extrazelluläre Matrix zur Verwendung in einem Verfahren zur Behandlung eines Subjekts mit einer ischämischen Gliedmaßenverletzung, wobei das Verfahren die Injektion von Fragmenten einer von Herzfibroblastenzellen abgeleiteten extrazellulären Matrix in ein Gewebe des Subjekts umfasst, das eine ischämische Gliedmaßenverletzung aufweist, wobei die Fragmente eine Dicke von 20-500 µm aufweisen und die Strukturproteine Fibronectin, Kollagen Typ I, Kollagen Typ III und Elastin umfassen, wobei 60 % bis 90 % der Strukturproteine, die in der manipulierten extrazellulären Matrix vorhanden sind, Fibronectin sind, wobei die aus Herzfibroblastenzellen gewonnene extrazelluläre Matrix fragmentiert und in einer isotonischen Lösung suspendiert wird, um eine injizierbare Suspension zu bilden, und dann dem Subjekten durch Injektion verabreicht wird,
wodurch der Schweregrad der ischämischen Gliedmaßenverletzung verringert wird.

17. Matrix zur Verwendung nach Anspruch 16, wobei die hergestellte extrazelluläre Herzmatrix ferner eines oder mehrere von Folgendem umfasst: latenter transformierender Wachstumsfaktor beta 1 (LTGFB-1) der matrizellulären Proteine, latenter transformierender Wachstumsfaktor beta 2 (LTGFB-2), Bindegewebswachstumsfaktor (CTGF), saures und cysteinreiches sekretiertes Protein (SPARC), Versican-Kernprotein (VCAN), Galectin 1, Galectin 3, Matrix-Gla-Protein (MGP), sulfatiertes Glykoprotein 1 und Biglycan.

18. Matrix zur Verwendung nach Anspruch 16 oder 17, wobei die künstlich hergestellte extrazelluläre Herzmatrix mit einer oder mehreren Zellen besiedelt ist, die therapeutisch für ischämische Gliedmaßenverletzungen sind, wobei optional die eine oder die mehreren Zellen, die für ischämische Gliedmaßenverletzungen therapeutisch sind, ausgewählt sind aus der Gruppe bestehend aus Skelettmyoblasten, embryonalen Stammzellen (ES) oder Derivaten davon, induzierten pluripotenten Stammzellen (iPS) oder Derivaten davon, multipotenten adulten Keimbahnstammzellen (maGCSs), mesenchymalen Stammzellen aus dem Knochenmark (BMSCs), sehr kleinen embryonalähnlichen Stammzellen (VSEL-Zellen), endothelialen Vorläuferzellen (EPCs), adipose-abgeleiteten Stammzellen, humanen mesenchymalen Stammzellen, die von iPS- oder ES-Zellen abgeleitet sind, humanen mesenchymalen Stromazellen, die von iPS- oder ES-Zellen abgeleitet sind, und Kombinationen davon.

19. Matrix zur Verwendung nach einem der Ansprüche 16-18, wobei das Verfahren ferner das Inkontaktbringen des Gewebes des Subjekts, das eine ischämische Verletzung aufweist, mit einem oder mehreren exogenen nicht-zellulären therapeutischen Mitteln umfasst, wobei optional das eine oder die mehreren nicht-zellulären therapeutischen Mittel einen oder mehrere Wachstumsfaktoren enthalten, wobei optional der eine oder die mehreren Wachstumsfaktoren ausgewählt sind aus der Gruppe bestehend aus stromal abgeleitetem Faktor-1 (SDF-1), epidermalem Wachstumsfaktor (EDF), transformierendem Wachstumsfaktor-α (TGF-α), Hepatozyten-Wachstumsfaktor (HGF), vaskulärem endothelialem Wachstumsfaktor (VEGF), von Plättchen abgeleitetem Wachstumsfaktor (PDGF), Fibroblasten-Wachstumsfaktor 1 (FGF-1), Fibroblasten-Wachstumsfaktor 2 (FGF-2), transformierendem Wachstumsfaktor-β (TGF-β) und Keratinozyten-Wachstumsfaktor (KGF).

20. Matrix zur Verwendung nach einem der Ansprüche 16-19, wobei die ischämische Gliedmaßenverletzung das Ergebnis von Atherosklerose ist.

## Revendications

1. Composition injectable pour traiter une maladie cardiaque, une lésion cardiaque ou une lésion ischémique, comprenant :
(a) des fragments d'une matrice extracellulaire dérivée de cellules de fibroblastes cardiaques modifiés (CF-ECM) comprenant des protéines structurelles fibronectine, du collagène de type I, du collagène de type III et de l'élastine, dans lesquels de 60 % à 90 % des protéines structurelles présentes dans la matrice extracellulaire est la fibronectine, l'un ou plusieur fragments étant suffisamment petits pour passer à travers l'ouverture d'injection d'une aiguille hypodermique de calibre 18 ; et
(b) un support injectable pharmaceutiquement acceptable, dans lequel les fragments sont en suspension dans le support.

2. Composition selon la revendication 1, dans laquelle les protéines structurelles de la matrice extracellulaire cardiaque modifiée ne sont pas chimiquement réticulées.

3. Composition selon la revendication 1 ou 2, dans laquelle l'un ou plusieurs fragments ont une épaisseur de 20 à 500 µm.

4. Composition selon l'une quelconque des revendications 1 à 3, dans laquelle l'un ou plusieurs fragments sont sous la forme d'une poudre lyophilisée.

5. Composition selon l'une quelconque des revendications 1 à 4, dans laquelle la matrice extracellulaire cardiaque modifiée comprend en outre une ou plusieurs des protéines matricielles, un facteur de croissance transformant latent bêta 1 (LTGFB-1), un facteur de croissance transformant latent bêta 2 (LTGFB-2), un facteur de croissance du tissu conjonctif (CTGF), une protéine sécrétée acide et riche en cystéine (SPARC), une protéine centrale versicane (VCAN), la galectine 1, la galectine 3, la protéine gla matricielle (MGP), la glycoprotéine sulfatée 1 et la biglycane.

6. Composition selon l'une quelconque des revendications 1 à 5, comprenant en outre un ou plusieurs agents thérapeutiques non cellulaires exogènes, éventuellement dans laquelle l'un ou plusieurs agents thérapeutiques non cellulaires exogènes comprennent un ou plusieurs facteurs de croissance, éventuellement dans laquelle l'un ou plusieurs facteurs de croissance sont sélectionnés dans le groupe constitué du facteur de croissance épidermique (EDF), du facteur de croissance transformant α (TGF-α), du facteur de croissance des hépatocytes (HGF), du facteur de croissance endothélial vasculaire (VEGF), du facteur de croissance dérivé des plaquettes (PDGF), du facteur de croissance des fibroblastes 1 (FGF-1), du facteur de croissance des fibroblastes 2 (FGF-2), du facteur de croissance transformant β (TGF-β), facteur dérivé du stroma 1 (SDF-1) et du facteur de croissance des kératinocytes (KGF).

7. Composition selon l'une quelconque des revendications 1 à 6, dans laquelle : (a) la composition est dépourvue de cellules fibroblastiques cardiaques intactes ; ou (b) la composition est exempte de cellules.

8. Composition selon l'une quelconque des revendications 1 à 6, comprenant en outre une ou plusieurs cellules qui sont thérapeutiques pour une maladie cardiaque, une lésion cardiaque ou une lésion ischémique, éventuellment dans laquelle l'une ou plusieurs cellules qui sont thérapeutiques pour une maladie cardiaque, une lésion cardiaque ou une lésion ischémique sont sélectionnées dans le groupe constitué des myoblastes squelettiques, des cellules souches embryonnaires (ES) ou de leurs dérivés, des cellules souches pluripotentes induites (iPS) ou de leurs dérivés, des cellules souches germinales adultes multipotentes (maGCS), des cellules souches mésenchymateuses de la moelle osseuse (BMSC), des très petites cellules souches de type embryonnaire (cellules VSEL), des cellules progénitrices endothéliales (EPC), des cellules cardiopoïétiques (CPC), des cellules dérivées de la cardiosphère (CDC), des cellules progénitrices cardiovasculaires *Isl1*+ multipotentes (MICP), des cellules progénitrices dérivées de l'épicarde (EPDC), des cellules souches adipeuses, des cellules souches mésenchymateuses humaines dérivées de cellules iPS ou ES, des cellules stromales mésenchymateuses humaines dérivées de cellules iPS ou ES, et des combinaisons de celles-ci.

9. Composition selon l'une quelconque des revendications 1 à 8, dans laquelle les fragments sont suffisamment petits pour passer à travers l'ouverture d'injection d'une aiguille hypodermique de calibre 27.

10. Composition selon l'une quelconque des revendications 1 à 9, destinée à être utilisée dans un procédé de traitement d'un sujet souffrant d'une maladie cardiaque, d'une lésion cardiaque, d'une maladie ischémique ou d'une lésion ischémique, le procédé comprenant l'injection au sujet d'une quantité efficace de la composition, grâce à quoi la gravité de la maladie cardiaque, de la lésion cardiaque, de la maladie ischémique ou de la lésion ischémique est diminuée.

11. Composition à utiliser selon la revendication 10, dans laquelle la maladie cardiaque, la lésion cardiaque, la maladie ischémique ou la lésion ischémique sont provoquées par un infarctus aigu du myocarde, une insuffisance cardiaque, une infection virale, une infection bactérienne, une anomalie congénitale, un accident vasculaire cérébral, un ulcère du pied diabétique, une maladie d'artère périphérique (MAP), un ulcère associé à la MAP ou une ischémie des membres.

12. Matrice extracellulaire dérivée de cellules de fibroblastes cardiaques modifiées sans cellules destinée à être utilisée dans un procédé de traitement d'un sujet souffrant d'une maladie ou d'une lésion ischémique, le procédé comprenant l'injection de la composition injectable selon l'une quelconque des revendications 1 à 6 dans un tissu du sujet qui est présentant une maladie ou une lésion ischémique, dans laquelle aucune cellule n'est ensemencée sur la matrice extracellulaire dérivée de cellules de fibroblastes cardiaques modifiés,
grâce à quoi la gravité de la maladie ou de la lésion ischémique est diminuée.

13. Matrice à utiliser selon la revendication 12, dans laquelle la matrice extracellulaire cardiaque artificielle acellulaire comprend en outre une ou plusieurs des protéines matricielles, du facteur de croissance transformant latent bêta 1 (LTGFB-1), du facteur de croissance transformant latent bêta 2 (LTGFB-2), du facteur de croissance du tissu conjonctif (CTGF), d'une protéine sécrétée acide et riche en cystéine (SPARC), d'une protéine centrale versicane (VCAN), de la galectine 1, de la galectine 3, de la protéine gla matricielle (MGP), de la glycoprotéine sulfatée 1 et de la biglycane.

14. Matrice à utiliser selon la revendication 12 ou 13, dans laquelle le procédé comprend en outre la mise en contact du tissu du sujet qui présente une maladie ou une lésion ischémique avec un ou plusieurs agents thérapeutiques non cellulaires exogènes ; éventuellement, l'un ou plusieurs agents thérapeutiques non cellulaires comprennent un ou plusieurs facteurs de croissance, éventuellement, l'un ou plusieurs facteurs de croissance sont sélectionnés dans le groupe constitué du facteur dérivé du stroma 1 (SDF-1), du facteur de croissance épidermique (EDF), du facteur de croissance transformant α (TGF-α), du facteur de croissance des hépatocytes (HGF), du facteur de croissance endothélial vasculaire (VEGF), du facteur de croissance dérivé des plaquettes (PDGF), du facteur de croissance des fibroblastes 1 (FGF-1), du facteur de croissance des fibroblastes 2 ( FGF-2), du facteur de croissance transformant β (TGF-β) et du facteur de croissance des kératinocytes (KGF).

15. Matrice à utiliser selon l'une quelconque des revendications 12 à 14, dans laquelle la maladie ou la lésion ischémique est provoquée par un infarctus du myocarde, un accident vasculaire cérébral, un ulcère du pied diabétique, une maladie artérielle périphérique (MAP), un ulcère associé à une PAD ou une ischémie d'un membre, éventuellement dans laquelle l'ischémie d'un membre est le résultat de l'athérosclérose ou du diabète.

16. Matrice extracellulaire dérivée de cellules de fibroblastes cardiaques modifiées destinée à être utilisée dans un procédé de traitement d'un sujet présentant une lésion ischémique d'un membre, le procédé comprenant l'injection dans un tissu du sujet qui présente une lésion ischémique d'un membre des fragments d'une matrice extracellulaire dérivée de cellules de fibroblastes cardiaques modifiées, les fragments ayant une épaisseur de 20 à 500 µm comprenant les protéines structurelles fibronectine, du collagène de type I, du collagène de type III et de l'élastine, dans laquelle de 60 % à 90 % des protéines structurelles présentes dans la matrice extracellulaire modifiée sont de la fibronectine, dans laquelle la matrice extracellulaire dérivée de cellules de fibroblastes cardiaques modifiées est fragmentée et mise en suspension dans une solution isotonique pour former une suspension injectable et est ensuite administrée au sujet par injection,
grâce à quoi la gravité de la lésion ischémique du membre est diminuée.

17. Matrice à utiliser selon la revendication 16, dans laquelle la matrice extracellulaire cardiaque modifiée comprend en outre une ou plusieurs des protéines matricielles, un facteur de croissance transformant latent bêta 1 (LTGFB-1), un facteur de croissance transformant latent bêta 2 (LTGFB-2), un facteur de croissance du tissu conjonctif (CTGF), une protéine sécrétée acide et riche en cystéine (SPARC), une protéine de base versican (VCAN), la galectine 1, la galectine 3, la protéine gla matricielle (MGP), une glycoprotéine sulfatée 1 et la biglycane.

18. Matrice à utiliser selon la revendication 16 ou 17, dans laquelle la matrice extracellulaire cardiaque modifiée est ensemencée avec une ou plusieurs cellules qui sont thérapeutiques pour une lésion ischémique d'un membre, éventuellement dans laquelle l'une ou plusieurs cellules qui sont thérapeutiques pour une lésion ischémique d'un membre sont sélectionnées parmi les groupe constitué de myoblastes squelettiques, de cellules souches embryonnaires (ES) ou de leurs dérivés, de cellules souches pluripotentes induites (iPS) ou de leurs dérivés, de cellules souches germinales adultes multipotentes (maGCS), de cellules souches mésenchymateuses de la moelle osseuse (BMSC), de très petites cellules souches embryonnaires (cellules VSEL), de cellules progénitrices endothéliales (EPC), de cellules souches adipeuses, de cellules souches mésenchymateuses humaines dérivées de cellules iPS ou ES, de cellules souches mésenchymateuses humaines des cellules stromales dérivées de cellules iPS ou ES, et des combinaisons de celles-ci.

19. Matrice à utiliser selon l'une quelconque des revendications 16 à 18, dans laquelle le procédé comprend en outre la mise en contact du tissu du sujet qui présente une lésion ischémique avec un ou plusieurs agents thérapeutiques non cellulaires exogènes, éventuellement dans laquelle l'un ou plusieurs agents thérapeutiques non cellulaires comprennent un ou plusieurs facteurs de croissance, l'un ou plusieurs facteurs de croissance étant éventuellement sélectionnés dans le groupe constitué du facteur dérivé du stroma 1 (SDF-1), du facteur de croissance épidermique (EDF), du facteur de croissance transformant α (TGF-α), du facteur de croissance des hépatocytes (HGF), du facteur de croissance endothélial vasculaire (VEGF), du facteur de croissance dérivé des plaquettes (PDGF), du facteur de croissance des fibroblastes 1 (FGF-1), du facteur de croissance des fibroblastes 2 (FGF-2), du facteur de croissance transformant -β (TGF-β) et du facteur de croissance des kératinocytes (KGF).

20. Matrice à utiliser selon l'une quelconque des revendications 16 à 19, dans laquelle la lésion ischémique d'un membre est le résultat de l'athérosclérose.
